Europäisches Patentamt

European Patent Office

Office européen des brevets

(19)

(11) **EP 1 645 881 A1**

(12) **EUROPEAN PATENT APPLICATION**

(43) Date of publication:
    **12.04.2006 Bulletin 2006/15**

(51) Int Cl.:
    *G01N 33/58* (2006.01)        *G01N 33/542* (2006.01)

(21) Application number: **04023709.1**

(22) Date of filing: **05.10.2004**

(84) Designated Contracting States:
    **AT BE BG CH CY CZ DE DK EE ES FI FR GB GR
    HU IE IT LI LU MC NL PL PT RO SE SI SK TR**
    Designated Extension States:
    **AL HR LT LV MK**

(71) Applicant: **DKFZ Deutsches
    Krebsforschungszentrum
    69120 Heidelberg (DE)**

(72) Inventors:
    • **Baudendiestel, Nina
      69221 Dossenheim (DE)**
    • **Langowski, Jörg
      69120 Heidelberg (DE)**

(74) Representative: **Isenbruck, Günter
    Isenbruck, Bösl, Hörschler, Wichmann, Huhn
    Patentanwälte
    Theodor-Heuss-Anlage 12
    68165 Mannheim (DE)**

(54) **Screening process for the detection and characterization of protein-protein-interactions in vivo by fluorescence cross correlation spectroscopy**

(57)    In a first aspect, the invention concerns a screening process for the detection of protein-protein-interactions in vivo comprising the following steps:

a) expressing a first fusion protein comprising a first protein that is fused with a first autofluorescent protein and expressing a second fusion protein comprising a second protein that is fused with a second autofluorescent protein in a cell, wherein the first and the second autofluorescent proteins of the fusion proteins differ from one another in at least one of their detectable photophysical parameters comprising different emission wavelengthes, different fluorescence life times and different polarisations of the emitted light of the autofluorescent proteins,

b) identifying the second protein that shows a protein-protein-interaction with the first protein by detecting coincidently occurring fluorescence signals of the first and the second autofluorescent proteins in the cells of step a), by measuring the intensities of the individual fluorescence signals of the first and of the second autofluorescent proteins, wherein the fluorescence signals of the first and of the second autofluorescent protein are differentiated from each other either by their different emission wavelengthes, by their different fluorescence life times or by the different polarization of the light emitted by those two autofluorescent proteins.

In a second aspect, the invention concerns the use of two individual autofluorescent proteins, which differ from each another in at least one detectable photophysical parameter, for detecting protein-protein interactions by fluorescence cross correlation spectroscopy (FCCS) in vivo. Hereby, the at least one detectable photophysical parameter which differs between the two individual autofluorescent proteins are either different emission wavelengthes, different fluorescence life times or different polarisations.

Figure 1

SEQUENCE LISTING

<110> Deutsches Krebsforschungszentrum (German Cancer Research Centre)

<120> Screening process for the detection and characterization of protein-protein-interactions in vivo by fluorescence cross correlation spectroscopy

<130> DK62957EP

<160> 16

<170> PatentIn version 3.1

<210> 1

<211> 225

<212> PRT

<213> Discosoma coral

<220>

<221> MISC_FEATURE

<222> (2)..(2)

<223> additional amino acid V in position 2 of DS-RED1 in comparison to mRED1

<400> 1

```
Met Val Arg Ser Ser Lys Asn Val Ile Lys Glu Phe Met Arg Phe Lys
1               5                   10                  15

Val Arg Met Glu Gly Thr Val Asn Gly His Glu Phe Glu Ile Glu Gly
            20                  25                  30

Glu Gly Glu Gly Arg Pro Tyr Glu Gly His Asn Thr Val Lys Leu Lys
            35                  40                  45

Val Thr Lys Gly Gly Pro Leu Pro Phe Ala Trp Asp Ile Leu Ser Pro
        50                  55                  60

Gln Phe Gln Tyr Gly Ser Lys Val Tyr Val Lys His Pro Ala Asp Ile
    65                  70                  75                  80
```

```
Pro Asp Tyr Lys Lys Leu Ser Phe Pro Glu Gly Phe Lys Trp Glu Arg
                85                  90                  95

Val Met Asn Phe Glu Asp Gly Gly Val Val Thr Val Thr Gln Asp Ser
            100                 105                 110

Ser Leu Gln Asp Gly Cys Phe Ile Tyr Lys Val Lys Phe Ile Gly Val
        115                 120                 125

Asn Phe Pro Ser Asp Gly Pro Val Met Gln Lys Lys Thr Met Gly Trp
    130                 135                 140

Glu Ala Ser Thr Glu Arg Leu Tyr Pro Arg Asp Gly Val Leu Lys Gly
145                 150                 155                 160

Glu Ile His Lys Ala Leu Lys Leu Lys Asp Gly Gly His Tyr Leu Val
                165                 170                 175

Glu Phe Lys Ser Ile Tyr Met Ala Lys Lys Pro Val Gln Leu Pro Gly
            180                 185                 190

Tyr Tyr Tyr Val Asp Ser Lys Leu Asp Ile Thr Ser His Asn Glu Asp
        195                 200                 205

Tyr Thr Ile Val Glu Gln Tyr Glu Arg Thr Glu Gly Arg His His Leu
    210                 215                 220

Phe
225


<210>  2

<211>  225

<212>  PRT

<213>  Discosoma coral


<220>

<221>  MISC_FEATURE

<222>  (2)..(2)

<223>  mutation site at position 2, preferably R (DS-RED1) is mutated to
       A (mRFP1)


<220>

<221>  MISC_FEATURE

<222>  (5)..(5)

<223>  mutation site at position 5, preferably K (DS-RED1) is mutated to
```

E (mRFP1)

<220>

<221>  MISC_FEATURE

<222>  (6)..(6)

<223>  mutation site at position 6, preferably N (DS-RED1) is mutated to D (mRFP1)


<220>

<221>  MISC_FEATURE

<222>  (21)..(21)

<223>  mutation site at position 21, preferably T (DS-RED1) is mutated to S (mRFP1)


<220>

<221>  MISC_FEATURE

<222>  (41)..(41)

<223>  mutation site at position 41, preferably H (DS-RED1) is mutated to T (mRFP1)


<220>

<221>  MISC_FEATURE

<222>  (42)..(42)

<223>  mutation site at position 42, preferably N (DS-RED1) is mutated to Q (mRFP1)


<220>

<221>  MISC_FEATURE

<222>  (44)..(44)

<223>  mutation site at position 44, preferably V (DS-RED1) is mutated to A (mRFP1)


<220>

<221>  MISC_FEATURE

<222>  (71)..(71)

<223>  mutation site at position 71, preferably V (DS-RED1) is mutated to A (mRFP1)

```
<220>

<221>  MISC_FEATURE

<222>  (83)..(83)

<223>  mutation site at position 83, preferably K (DS-RED1) is mutated
       to L (mRFP1)


<220>

<221>  MISC_FEATURE

<222>  (117)..(117)

<223>  mutation site at position 117, preferably C (DS-RED1) is mutated
       to E (mRFP1)


<220>

<221>  MISC_FEATURE

<222>  (124)..(124)

<223>  mutation site at position 124, preferably F (DS-RED1) is mutated
       to L (mRFP1)


<220>

<221>  MISC_FEATURE

<222>  (127)..(127)

<223>  mutation site at position 127, preferably V (DS-RED1) is mutated
       to T (mRFP1)


<220>

<221>  MISC_FEATURE

<222>  (150)..(150)

<223>  mutation site at position 150, preferably L (DS-RED1) is mutated
       to M (mRFP1)


<220>

<221>  MISC_FEATURE

<222>  (153)..(153)

<223>  mutation site at position 153, preferably R (DS-RED1) is mutated
       to E (mRFP1)


<220>
```

```
<221>  MISC_FEATURE

<222>  (156)..(156)

<223>  mutation site at position 156, preferably V (DS-RED1) is mutated
       to A (mRFP1)


<220>

<221>  MISC_FEATURE

<222>  (162)..(162)

<223>  mutation site at position 162, preferably H (DS-RED1) is mutated
       to K (mRFP1)


<220>

<221>  MISC_FEATURE

<222>  (163)..(163)

<223>  mutation site at position 163, preferably K (DS-RED1) is mutated
       to M (mRFP1)


<220>

<221>  MISC_FEATURE

<222>  (164)..(164)

<223>  mutation site at position 164, preferably A (DS-RED1) is mutated
       to R (mRFP1)


<220>

<221>  MISC_FEATURE

<222>  (174)..(174)

<223>  mutation site at position 174, preferably L (DS-RED1) is mutated
       to D (mRFP1)


<220>

<221>  MISC_FEATURE

<222>  (175)..(175)

<223>  mutation site at position 175, preferably V (DS-RED1) is mutated
       to A (mRFP1)


<220>

<221>  MISC_FEATURE

<222>  (177)..(177)
```

<223>  mutation site at position 177, preferably F (DS-RED1) is mutated
       to V (mRFP1)


<220>

<221>  MISC_FEATURE

<222>  (179)..(179)

<223>  mutation site at position 179, preferably S (DS-RED1) is mutated
       to T (mRFP1)


<220>

<221>  MISC_FEATURE

<222>  (180)..(180)

<223>  mutation site at position 180, preferably I (DS-RED1) is mutated
       to T (mRFP1)


<220>

<221>  MISC_FEATURE

<222>  (192)..(192)

<223>  mutation site at position 192, preferably Y (DS-RED1) is mutated
       to A (mRFP1)


<220>

<221>  MISC_FEATURE

<222>  (194)..(194)

<223>  mutation site at position 194, preferably Y (DS-RED1) is mutated
       to K (mRFP1)


<220>

<221>  MISC_FEATURE

<222>  (195)..(195)

<223>  mutation site at position 195, preferably V (DS-RED1) is mutated
       to T (mRFP1)


<220>

<221>  MISC_FEATURE

<222>  (197)..(197)

<223>  mutation site at position 197, preferably S (DS-RED1) is mutated
       to I (mRFP1)

<220>

<221> MISC_FEATURE

<222> (217)..(217)

<223> mutation site at position 217, preferably T (DS-RED1) is mutated to A (mRFP1)

<220>

<221> MISC_FEATURE

<222> (222)..(222)

<223> mutation site at position 222, preferably H (DS-RED1) is mutated to S (mRFP1)

<220>

<221> MISC_FEATURE

<222> (223)..(223)

<223> mutation site at position 223, preferably L (DS-RED1) is mutated to T (mRFP1)

<220>

<221> MISC_FEATURE

<222> (224)..(224)

<223> mutation site at position 224, preferably F (DS-RED1) is mutated to G (mRFP1)

<220>

<221> MISC_FEATURE

<222> (225)..(225)

<223> additional amino acid A at the C-terminus of mRFP1 in comparison to DS Red

<400> 2

```
Met Ala Ser Ser Glu Asp Val Ile Lys Glu Phe Met Arg Phe Lys Val
1               5                   10                  15

Arg Met Glu Gly Ser Val Asn Gly His Glu Phe Glu Ile Glu Gly Glu
            20                  25                  30

Gly Glu Gly Arg Pro Tyr Glu Gly Thr Gln Thr Ala Lys Leu Lys Val
```

```
                    35                      40                      45

        Thr Lys Gly Gly Pro Leu Pro Phe Ala Trp Asp Ile Leu Ser Pro Gln
            50              55                  60

        Phe Gln Tyr Gly Ser Lys Ala Tyr Val Lys His Pro Ala Asp Ile Pro
        65              70                  75                      80

        Asp Tyr Leu Lys Leu Ser Phe Pro Glu Gly Phe Lys Trp Glu Arg Val
                        85                  90                  95

        Met Asn Phe Glu Asp Gly Gly Val Val Thr Val Thr Gln Asp Ser Ser
                    100                 105                 110

        Leu Gln Asp Gly Glu Phe Ile Tyr Lys Val Lys Leu Arg Gly Thr Asn
                    115                 120                 125

        Phe Pro Ser Asp Gly Pro Val Met Gln Lys Lys Thr Met Gly Trp Glu
                130                 135                 140

        Ala Ser Thr Glu Arg Met Tyr Pro Glu Asp Gly Ala Leu Lys Gly Glu
        145                 150                 155                 160

        Ile Lys Met Arg Leu Lys Leu Lys Asp Gly Gly His Tyr Asp Ala Glu
                        165                 170                 175

        Val Lys Thr Thr Tyr Met Ala Lys Lys Pro Val Gln Leu Pro Gly Ala
                    180                 185                 190

        Tyr Lys Thr Asp Ile Lys Leu Asp Ile Thr Ser His Asn Glu Asp Tyr
                195                 200                 205

        Thr Ile Val Glu Gln Tyr Glu Arg Ala Glu Gly Arg His Ser Thr Gly
            210                 215                 220

        Ala
        225
```

<210>  3

<211>  35

<212>  DNA

<213>  Artificial sequence


<220>

<223>  oligonucleotide used for cloning

<400>  3
cttcgaattc tgatgatgtt ctcgggcttc aacgc                                    35

```
<210>  4
<211>  35
<212>  DNA
<213>  artificial sequence


<220>
<223>  oligonucleotide used for cloning
<400>  4
cggtggatcc cgcagggcca gcagcgtggg tgagc                                    35


<210>  5
<211>  35
<212>  DNA
<213>  artificial sequence


<220>
<223>  oligonucleotide used for cloning
<400>  5
cggctgcagg ccctgaagga ggagcctcag acagt                                    35

<210>  6
<211>  32
<212>  DNA
<213>  artificial sequence


<220>
<223>  oligonucleotide used for cloning
<400>  6
cggtggatcc cgaaatgttt gcaactgctg cg                                       32

<210>  7
<211>  34
<212>  DNA
<213>  artificial sequence


<220>
<223>  oligonucleotide used for cloning
<400>  7
```

aatttaccac cggtcatggc ctcctccgag gacg                                    34

<210>  8

<211>  36

<212>  DNA

<213>  artificial sequence


<220>

<223>  oligonucleotide used for cloning

<400>  8
taaattgcgg ccgctttagg cgccggtgga gtggcg                                  36

<210>  9

<211>  35

<212>  DNA

<213>  artificial sequence


<220>

<223>  oligonucleotide used for cloning

<400>  9
cttcgaattc tgatgatgtt ctcgggcttc aacgc                                   35

<210>  10

<211>  43

<212>  DNA

<213>  artificial sequence


<220>

<223>  oligonucleotide used for cloning

<400>  10
ataaattgga tcccgctctt cttcttctgg agataactgt tcc                          43

<210>  11

<211>  34

<212>  DNA

<213>  artificial sequence


<220>

<223>  oligonucleotide used for cloning

<400>  11
aattaacagt cgacgatggt gagcaagggc gagg                                        34


<210>  12

<211>  35

<212>  DNA

<213>  artificial sequence


<220>

<223>  oligonucleotide used for cloning

<400>  12
aatatatgga tcccgcttgt acagctcgtc catgc                                       35


<210>  13

<211>  34

<212>  DNA

<213>  artificial sequence


<220>

<223>  oligonucleotide used for cloning

<400>  13
aattaacagt cgacgatggc ctcctccgag gacg                                        34


<210>  14

<211>  36

<212>  DNA

<213>  artificial sequence


<220>

<223>  oligonucleotide used for cloning

<400>  14
aatttatgga tcccgttagg cgccggtgga gtggcg                                      36


<210>  15

<211>  21

<212>  DNA

<213>  artificial sequence

<220>

<223>  oligonucleotide used for cloning

<400>  15
gaattcgagc tcgcccgggg a                                                    21

<210>  16

<211>  18

<212>  DNA

<213>  artificial sequence

<220>

<223>  oligonucleotide used for cloning

<400>  16
gtactaacca ggcccgac                                                        18

**Description**

In a first aspect, the invention concerns a screening process for the detection of protein-protein-interactions in vivo comprising the following steps:

a) expressing a first fusion protein comprising a first protein that is fused with a first autofluorescent protein and expressing a second fusion protein comprising a second protein that is fused with a second autofluorescent protein in a cell, wherein the first and the second autofluorescent proteins of the fusion proteins differ from one another in at least one of their detectable photophysical parameters comprising different emission wavelengthes, different fluorescence life times and different polarisations of the emitted light of the autofluorescent proteins,

b) identifying the second protein that shows a protein-protein-interaction with the first protein by detecting coincidently occurring fluorescence signals of the first and the second autofluorescent proteins in the cells of step a), by measuring the intensities of the individual fluorescence signals of the first and of the second autofluorescent proteins, wherein the fluorescence signals of the first and of the second autofluorescent protein are differentiated from each other either by their different emission wavelengthes, by their different fluorescence life times or by the different polarization of the light emitted by those two autofluorescent proteins.

Hereby, the first protein of the first fusion protein may be the so called "bait protein" (that means the protein interaction partners shall be identified for) and the second protein of the second fusion protein may be the so called "prey protein" (that means those proteins interaction partners shall be identified from) or vice versa. Preferably more than one prey protein is hereby used, more preferably the more than one prey proteins consist of the individual proteins of a protein library, whereby each of the individual "prey protein"-containing fusion proteins is expressed together with the "bait-protein" containing fusion protein in an individual cell clone.

In step a) the first and the second autofluorescent proteins of the fusion proteins preferably differ from each another in their different emission wavelengthes and in step b) the intensities of the individual fluorescence signals of the first and the second autofluorescent proteins in the cells of step a) are detected preferably by measuring the fluorescence signals at these different emission wavelengthes.

Hereby, one of the first and the second autofluorescent proteins is preferably a green autofluorescent protein and the other of the first and the second autofluorescent proteins is preferably a red autofluorescent protein.

The red autofluorescent protein is preferably a dimer or monomer, more preferably a dimeric or monomeric derivate of the protein DS-RED1 as set force in SEQ ID No. 1, more preferably a monomeric derivate of DS-RED1 as set force in SEQ ID No. 1 comprising non-silent mutations at the positions N42, V44, K163, V175, F177 and S197, T217, more preferably a monomeric derivate of DS-RED1 that additionally comprises non-silent mutations at the positions R2, K5, N6, I125, V127,I180, R153, H162, A164, L174, Y192, Y194, H222, L223, F224, L225 and particularly the monomeric derivate of DS-RED1 called mRFP1 as set force in SEQ ID No. 2.

In a second aspect, the invention concerns the use of two individual autofluorescent proteins, which differ from each another in at least one detectable photophysical parameter, for detecting protein-protein interactions by fluorescence cross correlation spectroscopy (FCCS) in vivo. Hereby, the at least one detectable photophysical parameter which differs between the two individual autofluorescent proteins are either different emission wavelengthes, different fluorescence life times or different polarisations. In particular the invention is related to the use of a green autofluorescent protein and of the red autofluorescent protein mRFP1 for detecting protein-protein interactions by fluorescence cross correlation spectroscopy (FCCS) in vivo.

Protein-protein-interactions have emerged as a critical theme in biochemistry in the postgenomic era. With an increasing number of novel proteins revealed by genomic research and the subsequent task of characterizing their functions, various new technologies have been developed for in vivo applications and high-throughput-screenings.

One of the screening methods for studying protein-protein-interactions known to a person skilled in the art is the yeast two-hybrid system. In the yeast two-hybrid system the two functional domains of the GAL4 transcription factor, the DNA-binding domain and the transactivation domain, are both expressed in a specific yeast strain as two separate fusion proteins. The first fusion protein comprises the GAL4 DNA-binding domain linked to the "bait"-protein (the protein interaction partners need to be identified for) and the second fusion protein comprises the GAL4 transactivation domain linked to the individual proteins of a protein library (the so-called "prey"-proteins). The first fusion bearing the GAL4 DNA binding domain can bind to the promoter sequence (the "upstream activating sequence") of a β-galactosidase reporter gene in the yeast strain. Whenever the bait-protein of the first fusion protein is able to interact with a specific library protein (prey protein) the GAL4 DNA binding domain of the first fusion protein will get into close proximity to the GAL4 transactivation domain in the second fusion protein and this will lead to the activation of the β-galactosidase reporter gene expression. Thus, the yeast colonies expressing a first and a second fusion protein with interacting bait and prey proteins will turn blue in a β-galactosidase test and therefore will be identified. Finally the interacting library protein (prey protein) can be isolated as fusion protein with the GAL4 transactivation domain from the yeast colony that had been "positive" in the β-galactosidase test.

However, in the yeast two hybrid system usually also many "false positive" colonies are identified, as for instance those colonies expressing a second fusion protein comprising a library protein that is an intact transcription factor itself and that therefore is capable to activate the reporter gene expression on its own. Another disadvantage of this system might be its performance in yeast cells instead of human cells, because it is still unclear whether human proteins expressed in yeast cells are subjected to the same posttranslational modifications and will perform the same 3-dimensional folding as they perform in human cells. Therefore protein-protein-interactions that can be observed in human cells might not necessarily be detected in a screening method for detecting protein-protein-interactions in yeast.

One of the most promising spectroscopic tools for studying protein-protein-interactions in the living cell, fluorescence resonance energy transfer (FRET), has recently been used for the development of efficient intracellular ligand-bindung (Miyawaki et al., Nature, 388, 882-887, 1997) and protease assays (Tawa et al., Cell Death Differ., 8, 30-37, 2001). Here, cellular processes can be evidenced by spectral changes of the emission signal because of distance-dependent energy transfer from an excited donor dye to a long-wavelength acceptor dye. For the establishment of a screening assay for detecting protein-protein-interactions the bait protein may be coupled to a short-wavelength donor dye and the prey protein may be coupled to a long-wavelength acceptor dye. Whenever an interaction between bait and prey protein occurs, the two dyes get into close proximity to one another. When the short-wavelength donor dye is excited, the excitation state can be transfered to the long-wavelength acceptor dye and a spectral change of the emission signal will occur and serve as a detection signal of an interaction between the bait and the prey protein. Because of its high specifity, FRET has proven useful to investigate diverse molecular interactions and conformational changes even on a single-molecule scale (Weiss et al., Science 283, 1676-1683, 1999; Selvin et al., Nat. Struct. Biol., 7, 730-734, 2000). However, because FRET requires spatial distances between donor and acceptor dyes of typically 20-60 Angstrom, this imposes a fundamental limit on probing molecular interactions. In particular, larger bait- and prey-proteins or bait- and prey-complexes will be difficult to detect by FRET, since in this case the donor and acceptor dyes will not get into a proximity that is close enough for a fluorescence resonance energy transfer to occur. Additionally FRET can only occur when the fluorescence emission spectrum of the donor molecule overlaps with the adsorption spectrum of the acceptor molecule. Furthermore, the transition dipole of the donor and acceptor molecules have to be oriented approximately parallell for FRET to occur which might not always be the case. Therefore the possibilities to use FRET for detecting protein-protein interactions in a reliable assay are quite limited.

Another technique for studying molecular movements or interactions is fluorescence autocorrelation spectroscopy (FCS), whereby fluctuations in the fluorescence signal from fluorescently labelled molecules inside the small confocal volume are monitored. These fluctuations depend on the number of fluorophores (or molecules) in the detection volume - and in so far on the concentration of the solution - and on the mass of the molecules that influence diffusion parameters. Therefore FCS can be used for the measurement of concentrations or of any increase in the mass of biomolecules (i.e. protein), for instance as a result of an interaction with a second biomolecule (i.e. protein), which can be detected as an increase in the particle's diffusion time. But since the FCS signal is influenced by the concentration of the fluorescent molecule as well as by molecule masses or diffusion parameters, assays for detecting protein-protein interactions by measuring fluorescence autocorrelations (FCS) are rather error-prone.

The detection of protein-protein-interactions by measuring dual colour fluorescence cross correlation spectroscopy (dsFCCS) evades the aforementioned limitations by recording coincident signal fluctuations from a confocal detection volume in two spectrally distinct emission channels which are derived from the bait and the prey proteins, whereby the bait and the prey proteins are both tagged to a different-coloured fluorescent protein. Hereby, the emission spectra of the two different coloured fluorescent proteins should show the lowest overlap possible, and preferably show no overlap at all. Moreover, dcFCCS allows for probing of extremely low fluorophore concentrations with a temporal resolution down to tenth of nanoseconds. In a single-molecule regime, coincident signal fluctuations in both detection channels unambiguously indicate the presence of tight chemical or physical linkages between the fluorophores and therefore spatial proximities between two proteins produced by an interaction can be detected by an FCCS signal. Previously, the technique of dcFCCS has been successfully used to quantitatively study endonucleotytic cleavage of double-labeled double-stranded DNA (dsDNA) in real time (Kettling et al., PNAS, 95, 1416-1420; Heinze et al., PNAS, 97, 10377-10382). By limiting readout parameters to either cross correlation amplitudes or coincidence measurements, the speed of the assay could be improved to meet high-throughput screening standards enabling reliable yes-or no assessments within subsecond sampling times (Koltermann et al., PNAS, 95, 1221-1426).

However, in all methods making use of dsFCCS for the detection of protein-protein-interactions known so far, at least one of the proteins in question (the bait and/or the prey fusion protein) had been linked to a chemical dye (instead of being linked to fluorescent proteins). Thus, these methods are usually performed in vitro.

For instance, in Doi et al., Genome Research, 12; 487-492 (2002) a high-throughput assay for the **in vitro** analysis of **DNA-protein interactions** using FCCS with **chemical dyes** is disclosed. In this assay the interaction between the promoter region of the DNA that binds to the transcription factor AP-1 and the transcription factor AP-1 consisting of the two components Fos and Jun had been determined by FCCS. Whenever Fos was rhodamine-labelled by in-vitro translation and the DNA promoter region was labelled by Cy5 in the presence of unlabeled Jun, a DNA-protein interaction could be detected by FCCS ([Cy5]-DNA interacts with [Rhodamine]-Fos/Jun, an FCCS-signal was measured). But whenever a rhodamine-labelled Fos and a Cy5-labeled DNA promoter region had been tested in the presence of unlabelled Fos (instead of Jun), no interaction could be detected by FCCS ([Cy5]-DNA does not interact with [Rhodamine]-Fos/Fos, no FCCS-signal was observed).

This use of proteins linked to chemical dyes for the performance of protein-protein-interactions-assays by FCCS as well as the usual in vitro performance of FCCS-based protein-protein interaction assays according to the state of the art has various disadvantages:

- the use of chemical dyes usually can not be combined with an in vivo assay (since in this case it would be unclear how the chemical dye can be introduced into a living cell and how the dye will be chemically linked to a specific protein in the living cell)

- protein-protein-interactions observed in "in vitro-assays" might substantially differ from those protein-protein-interactions that occur "in vivo" (i.e. because of different 3-dimensional foldings in vitro and in vivo)

- in vitro-assays using chemical dyes are more expensive, more time-consuming and therefore less suitable for the performance of high throughput-screens than in vivo-assays using "cloneable fluorescent protein tags" (e.g. EGFP etc.).

However, so far the development of screening assays using "cloneable fluorescent protein tags" for detecting protein-protein-interactions by FCCS in vivo has been limited by the absence of suitable pairs of fluorescent proteins that fulfil the necessary criteria for the FCCS-assay, i.e. largely different emission spectra, but similar photostabilities (to allow FCCS) and a non-toxicity of the fluorescent fusion proteins or fluorescent fusion protein aggregates to the cell (to allow an in vivo application).

In Kohl et al., PNAS, 99, No. 19, 12161-12166 a combined FRET/FCCS **in vitro-protease assay** that is based on red-shifted GFP and the red fluorescent protein DS-RED, both connected to each other by a short protein linker of 32 amino acids length containing a recombinant fragment from tobacco etch virus (TEV) protease, is disclosed. On protease cleavage, the protease-dependent cleavage between the two fluorescent proteins in the reporter fusion protein was monitored quantitatively by using FCCS, which allows for the discrimination of different protease concentrations within the nanomolar range. FRET efficiency of the substrate was simultaneously observed during the protease digest by recording the molecular photon yield per second, providing an alternative means to monitor the cleavage reaction. In this protease assay the reporter protein, comprising two different fluorescent proteins linked by a protease cleavage site, first was expressed in bacteria, then the bacteria were lysed, the reporter protein was purified and isolated by affinity chromatography and then monitored quantitatively by FCCS and FRET during protease incubation, whereby the protease concentration could be detected. Thus, the protease assay disclosed in Kohl et al. - although using a pair of "cloneable" fluorescent proteins instead of chemical dyes - is designed as a classical "in vitro assay" which - as previously mentioned - is clearly more expensive and time-consuming than an in vivo assay. The design of the protease assay as "in vitro assay" had obviously been necessary, because the reporter protein, the cleavage products of the reporter protein or aggregates formed by any of those proteins in the cell might have been cytotoxic to the cell.

In particular, fluorescent proteins which are oligomeric (that means which consist predominantly of tetramers, trimers, certain dimers etc.) and/or which tend to form large aggregates in cells are often highly cytotoxic to living cells. Therefore fluorescent proteins that are oligomeric are generally not suitable for expressing fusion proteins comprising a fluorescent protein linked to an other protein in living cells.

For the combination with the non-toxic EGFP protein (with a green fluorescence) for in-vivo dual color fluorescence cross correlation spectroscopy there is a strong need to provide a red fluorescent protein, since red and green fluorescent proteins show a minimum overlap of their emission spectra. But unfortunately, all red fluorescent proteins that are commercially available so far (DsRED1, DsRED2, hcRED1) show a strong tendency to form oligomers or aggregates, are therefore cytotoxic and in general not suitable for in-vivo applications (see page 79, section 5.3 of Bacia et al., Methods, 29, 74-85 (2003)). Particularly, the native dsRED1 protein (also called drFP583), a red fluorescent protein cloned from *Discosoma coral,* that was used for the in vitro protease assay in Kohl et al., shows a strong tendency to aggregate (the native DsRED1 protein is tetrameric, see Kohl et al., PNAS, 99, No. 19, 12161-12166 and Campbell et al., PNAS, 99, No. 12, 7877-7882). In so far the protease assay disclosed in Kohl et al. had to be performed in vitro.

In Campbell et al., PNAS, 99, No. 12, 7877-7882 (2002) a monomeric mutant of DsRED1, which is called mRFP1, has been disclosed. But in Campbell et al. it is also discussed that mRFP1 possesses many unfavourable physical properties (in particular a very low extinction coefficient and a low fluorescence quantum yield), which limit the brightness of fully mature mRFP 1 to approximately 25 % of the brightness of Ds-RED 1.Therefore Campbell et al. render mRFP 1 currently nonoptimal for the construction of FRET-based sensors that could be used for detecting protein-protein interactions by dcFCCS. Instead, Campbell et al. propose to engineer more desireable mRFP1-variants with higher quantum yields and a diminished green component in the future to provide new FRET-based sensors (see the last column of Campbell et al., PNAS, 99, No. 12, 7877-7882 (2002)). The use of mRFP1 for dual color fluorescence cross correlation spectroscopy is not even discussed or suggested in Campbell et al..

The monomeric structure of mRFP 1 has also been mentioned in Bacia et al., Methods, 29, 74-85 (2003) (on page 79, chapter 5.3). But also according to Bacia et al. mRFP1 mRFP1 is not a suitable fluorescent protein for measuring fluorescence autocorrelation signals (FCS), since the extinction coefficient and quantum yield of mRFP1 is extraordinarily low and since its photobleaching rates are high. Again, the use of mRFP for measuring cross correlation signals (deFCCS) has not even been discussed or suggested by Bacia et al.. Instead, it is discussed in Bacia et al. on page 83 in chapter 9.3 that strong photobleaching (as observed for mRFP1) represents a mayor artefact source for dcFCCS.

It is the task of the present invention to provide a screening method for detecting protein-protein-interactions which overcomes the aforementioned disadvantages of the screening methods for detecting protein-protein-interactions as known in the prior art, such as being in vitro assays or the use of expensive chemical dyes. In particular it is the task of the present invention to identify a "cloneable", non-cytotoxic, red fluorescent protein that can be used in combination with the green fluorescent protein (in particular with EGFP) for detecting protein-protein-interactions by measuring dual-color fluorescence cross correlation spectroscopy (dcFCCS) in vivo.

These tasks are solved by a screening process for the detection of protein-protein-interactions in vivo comprising the following steps:

a) expressing a first fusion protein comprising a first protein that is fused with a first autofluorescent protein and expressing a second fusion protein comprising a second protein that is fused with a second autofluorescent protein in a cell, wherein the first and the second autofluorescent proteins of the fusion proteins differ from one another in at least one of their detectable photophysical parameters comprising different emission wavelengthes, different fluorescence life times and different polarisations of the emitted light of the autofluorescent proteins,

b) identifying the second protein that shows a protein-protein-interaction with the first protein by detecting coincidently occurring fluorescence signals of the first and the second autofluorescent proteins in the cells of step a), by measuring the intensities of the individual fluorescence signals of the first and of the second autofluorescent proteins, wherein the fluorescence signals of the first and of the second autofluorescent protein are differentiated from each other either by their different emission wavelengthes, by their different fluorescence life times or by the different polarization of the light emitted by those two autofluorescent proteins.

Hereby, the first protein that is fused with a first autofluorescent protein is preferably the so-called "bait protein", representing that protein, interaction partners have to be found for, whereas the second protein that is fused with the second autofluorescent protein is the so-called "prey-protein".

In a preferred embodiment of the process, there are more than one "prey-proteins", that means more than one second proteins which are all fused to the second autofluorescent protein. It is further preferred that the more than one prey proteins consist of the individual proteins of a protein library, wherein each of these library proteins are fused with the second autofluorescent protein which differs from the first autofluorescent protein by at least one of the following detectable photophysical parameters: its fluorescence emission wavelength, its fluorescence life time and/or the polarisation of the light emitted by this autofluorescent protein.

The expression of the first and of the second fusion protein in a cell according to step a) usually comprises the introduction of one or more expression vectors into the cell, wherein these vectors contain the DNA coding for the first and the second fusion proteins which are cloned into the expression cassettes of those vectors under the control of their respective promoters. The introduction of the one or more expression vectors into the cells can be done for instance by microinjection or preferably by transformation of the cells. The transformation of the cells with the one or more expression vectors is usually done by methods known to persons skilled in the art, wherein calcium chloride, rubidium chloride, lithium chloride, calcium phosphate, lipids, liposomes are applied or by using the methods also known to persons skilled in the art of lipofection, infection with viruses or virus-particles, particle bombardment ("gene gun" method), heat shock transformation, electroporation or combinations thereof.

The cell can be transformed transiently or permanently with the expression vector. After the performance of a transient transformation the introduced expression vector will remain in the cell independent from the genome, that means the expression vector sequences (including the fusion protein sequences) will not be integrated into the genome of the cell. Upon division of the transiently transformed cells, the expression vector will not be dublicated and will not be transferred to the daughter cells. Therefore the transiently transformed cells will "lose" the introduced expression vectors after some cell cycles, since the cells containing expression vectors become more and more "diluted" by cells without expression vectors after several cell divisions. Alternatively, the cell can be transformed permanently. But also in transiently transformed cells the loss of the expression vector can be avoided by applying a selection pressure on the presence of cells containing the expression vector, if expression vectors with appropriate selection markers are used.

After the performance of a permanent transformation the expression vector (including the fusion protein sequences) will be integrated into the genome of the cell. Upon division of the permanently transformed cells, the complete genome including the integrated expression vector will be replicated and will be transferred to the daughter cells.

It is also possible that only one vector containing either the bait fusion or the prey fusion protein is permanently transformed into the cell and that the other fusion protein is transiently transformed into the cell.

Transient and permanent transformation techniques are known to a person skilled in the art and can be learned from standard literature (Freshney, IR; Culture of Animal Cells, 2000, 4th Ed. Wiley-Liss).

The cells of step a) can be prokaryotic cells, i.e. bacteria, but are preferably eukaryotic cells, i.e. yeast cells, eukaryotic cell cultures (cell lines), eukaryotic primary cultures, in particular human cell lines as HeLa cells, HEK 293 cells etc..

The choice of the expression vector(s) for the expression of the first and second fusion proteins of step a) is usually strongly influenced by the choice of cells to be transformed, i.e. for the expression in eukaryotic cells also eukaryotic expression vectors have to be used, for the expression in yeast cells yeast expression vectors have to be used etc. Generally all kinds of expression vectors known to a person skilled in the art can be used for the expression of the first and the second fusion proteins of step a), for instance bacterial expression vectors, plasmids, bacteriophages and preferably eukaryotic expression vectors, particularly human expression vectors. These expression vectors can generally comprise all kinds of promoters known to a person skilled in the art. The choice of the promoter is also strongly influenced by the choice of cells to be transformed, that means when eukaryotic cells are transformed, then also eukaryotic promoters, as for instance the CMV promoter or the SV-40 promoter, will be used.

In step b) the second protein of the second fusion protein (the "prey protein") that interacts with the first protein of the first fusion protein (the "bait protein") is identified by detecting coincidently occurring fluorescence signals of the first and the second autofluorescent proteins in the cells of step a). Hereby, the intensities of the individual fluorescence signals of the first and the second autofluorescent proteins are first measured and set in relation to each other afterwards.

For this purpose the individual fluorescence signals of the first and of the second autofluorescent protein need to be distinguishable from each other. This discrimination between the fluorescence signal of the first autofluorescent protein and of the second autofluorescent protein can be performed by various detection methods that depend on the photophysical properties of the first and the second autofluorescent proteins which are used.

If the light which is emitted either by the first or by the second autofluorescent protein shows distinguishable polarisations, then it is possible to discriminate between the individual fluorescence signals derived from the first or from the second autofluorescent protein by measuring the individual intensities of the fluorescence emission signals showing either the polarization of the first autofluorescent protein or showing the polarization of the second autofluorescent protein. In this case, the fluorescence emitted from the cells of step a) is detected in two individual modules which are both able to detect only light with one specific polarisation (either with the polarisation of the fluorescence emitted by the first autofluorescent protein or with the polarisation of the fluorescence emitted by the second autofluorescent protein).

It is also possible to discriminate between the individual fluorescence signals derived from the first or from the second autofluorescent protein by associating the individual fluorescence signals to their individual fluorescence life times. This detection method can be applied, whenever a first and of a second autofluorescent protein are used which differ from each other in their fluorescence life times. In this case, the fluorescence emitted from the cells of step a) is detected in two individual modules which both detect fluorescent light with only one specific fluorescence life time (either with the fluorescence life time that is characteristic for the fluorescence emitted by the first autofluorescent protein or with the fluorescence life time that is characteristic for the fluorescence emitted by the second autofluorescent protein).

In a preferred embodiment of the process of the invention the first and the second autofluorescent proteins have different fluorescence emission wavelengthes and therefore the individual fluorescence signals derived from the first and from the second autofluorescent protein can be easily distinguished from each other by spectral discrimination. Hereby, the fluorescence emitted from the cells of step a) is detected in two individual modules which both detect fluorescent light with only one specific fluorescence emission wavelength (either with the fluorescence emission wavelength that is characteristic for the fluorescence emitted by the first autofluorescent protein or with the fluorescence emission wavelength that is characteristic for the fluorescence emitted by the second autofluorescent protein).

Since in step b) the coincidently occurring fluorescence signals of the first and of the second autoffuorescent protein (the so called fluorescence cross correlation signals (FCCS-signals)) are detected, either by discriminating between the two fluorescence signals by their different fluorescence emission wavelengthes (spectral discrimination), by their different fluorescence life times (life time discrimination) or by the different polarisation of the fluorescent light emitted by them (discrimination by different polarisations), it is crucial that the two different fusion proteins comprising the two different autofluorescent proteins are expressed more or less at the same level, so that the two fluorescent emission signals will have similar intensities.

Thus, in a preferred embodiment of the process of the invention, the first and the second fusion protein will be expressed under the control of identical promoters, for instance by cloning DNA coding for the first and second fusion protein each into an individual expression vector, whereby the expression vectors comprise the identical promoters. Preferably, the first and the second fusion protein each will be expressed by an individual expression vector, whereby the two expression vectors are identical (with exception of the DNA coding for the first or second fusion protein in the multiple cloning site). It is also preferred that the first and the second fusion protein each are expressed under the control of the same promoter in the same expression vector. Hereby, preferably expression vectors are used which contain an expression cassette consisting of a promoter, followed by the DNA sequence coding for the first fusion protein, followed by an IRES-element (internal ribosomal entry site), followed by the DNA sequence coding for the second fusion protein. In this case a bicistronic mRNA will be produced which comprises the mRNA of the first and the second fusion protein on a single RNA strand, but leads to the production of more or less identical amounts of two individual fusion proteins. Preferred is also the use of bidirectional expression vectors with bidirectional expression cassettes, whereby in one direction the DNA coding for the first fusion protein is cloned and in the other direction the DNA coding for the second fusion protein is cloned, both under control of the same promoter. An example for such a preferred bidirectional expression vector are the bidirectional Tet Expression Vectors (i.e. the pBI-vector from Clontech).

After their transformation with expression vector(s) comprising the first and the second fusion proteins the cells of step a) are plated on culture dishes or on cover slips, where the cells need to attach themselves to the bottom of the dish or to the cover slip for fixation. Some cells, like for instance the human cell line HeLa, are self-adherent to plastic surfaces, as i.e. cover slips or the bottom of culture dishes. Other cells which are not self-adherent need a layer of another sort of adherent cells (so called "feeder cell" layer, which can be for instance fibroblast cells). These cells will attach themselves to the feeder cell layer, whereby the feeder cells themselves are self-adherent to the plastic surface. In this way the non-adherent cells can be fixed to the cover slip or dish bottom. Other cells need cover slips or culture dishes which have been coated before with specific substances, as for instance gelatine, to adhere themselves to the plastic surface.

Then the cells are incubated at culture conditions that are appropriate for the respective cell type that is used. For example HeLa cells are incubated in a 5 % $CO_2$ humidified atmosphere at 37 °C and passaged in RPMI 1640 medium (without phenol red) from Invitrogen Life Technologies supplemented with 10 % fetal calf serum (Invitrogen Life Technologies). The incubation time that is sufficient for allowing the expression and full maturation of the fusion proteins (both tagged with fluorescent proteins) is between 12 hours and 48 hours, preferably between 24 hours and 36 hours, particularly 30 hours after that incubation time the in vivo measurement of the FCCS-signal can be performed.

For the measurement the cover slips or culture dishes which are preferably cultured sub-confluent are mounted on the scanning stage of the FCCS unit in an incubator chamber at 37°C. Fluorescence auto correlation signals (FCS-signals) and fluorescence cross correlation signals (FCCS-signals) can be measured for example by a Fluorescence Fluctuaction Microscope (FFM) which is constructed as described in Wachsmuth, J. Mol. Biol., 298, 677-689 (2000). This setup combines a FCS/FCCS module and a galvanometer mirror scanning unit attached to the video port of an inverted IX-70 microscope (Olympus, Hamburg, Germany) with a Uplan Apo 60X, 1.2 NA water immersion objective or by other setups which are known to a person skilled in the art and/or which are commercially available.

In another preferred embodiment of the process of the invention, the fluorescence cross correlation signal of the cells of step a) is determined by comparing the originally measured fluorescence cross correlation signal with the corresponding cross correlation signal measured for cells, which had been transformed with a negative control vector, indicating 0 % protein-protein-interaction, and by comparing the measured fluorescence cross correlation signal with the corresponding cross correlation signal measured for cells, which had been transformed with a positive control vector, indicating 100 % protein-protein-interaction. The structure of the negative and positive control vectors comprising the DNA coding for negative or positive control construct strongly depend on the applied detection method (detection by spectral discrimination, fluorescence life time discrimination or discrimination by different polarization of the emitted light).

In step b) of the screening process the second protein (the prey protein) which shows a protein-protein-interaction with the first protein (the bait protein) is identified by determining the fluorescence cross correlation signal of the cells of step a), that means by detecting the coincidently occurring fluorescence signals of the first and of the second autofluorescent proteins, wherein the fluorescence signals of the first and of the second autofluorescent proteins can be distinguished by the above-mentioned detection methods. Hereby, the detection method of spectral discrimination between the fluorescence signals of the first and of the second autofluorescent proteins is preferred.

It is particularly important that the two applied autofluorescent proteins have photobleaching rates which are significantly smaller than 1000 $s^{-1}$ at the usually applied laser intensity of approximately 0,1 - 10 kWatt/cm$^3$, since the diffusion time of an average protein in a living cell in the range of 1 ms. Whenever the life time in the focus becomes smaller than the diffusion time, FCS measurements will be obtained that are strongly error-prone or even unusable. Therefore merely such autofluorescent proteins can be applied for the screening method of the invention that show very low photobleaching rates, so that the life time in the focus will be significantly higher than the diffusion time. Thus, the autofluorescent proteins have photobleaching rates which are significantly smaller than 1000 $s^{-1}$, preferably smaller than 10 $s^{-1}$, particularly smaller than 1 $s^{-1}$.

Moreover, it is particularly preferred that the fluorescence emission wavelengthes of the applied first and of the applied second autofluorescent proteins show a maximum difference from each other (that means they show a minimum spectral overlap). For instance, the difference in the fluorescence emission wavelengthes is particularly large for green autofluorescent and red autofluorescent proteins or for yellow autofluorescent and blue autofluorescent proteins. Unfortuneately the blue fluorescent derivates of EGFP, such as EBFP and ECFP, usually show a strong photobleaching.

Thus, in a preferred embodiment of the screening process one of the two fusion proteins comprises a green autofluorescent protein and the other of the two fusion proteins comprises a red autofluorescent protein. In this embodiment of the screening process the green autofluorescent protein is preferably GFP, a green fluorescent protein derived from the jellyfish *Aequorea victoria,* or some other green fluorescent GFP-derivate, such as EGFP etc. The red autofluorescent protein has preferably the structure of a dimer, or more preferably of a monomer, since oligomeric structures are often cytotoxic to the cell.

In this preferred embodiment of the screening process the red autofluorescent protein is a dimeric - or more preferably a monomeric - derivate of Ds-RED as set force in SEQ ID No. 1.

A "derivate" of Ds-RED as set force in SEQ ID No. 1 is defined as a protein having basically the amino acid sequence as set force in SEQ ID No. 1, but additionally comprising more than 5, preferably more than 8, more preferably more than 12, more preferably more than 20, in particular 33 non-silent point mutations at any of the mutation sites mentioned in SEQ ID No. 2, wherein these point mutations decrease the oligomeric state of Ds-RED (as set force in SEQ ID No. 1) and/or influence the physical properties of the fluorophore, i.e. support the fluorescence or photostability of the protein and/or influence the rate of maturation.

Dimeric - or more preferably monomeric - derivates of Ds-RED as set force in SEQ ID No. 1 were isolated by producing a Ds-RED mutation library by error prone PCR. Error prone PCR is a random mutagenesis technique for introducing amino acid changes into proteins (Campbell et al., PNAS, 99, No. 12, 7877-7882). Hereby, the mutations are deliberately introduced during PCR through the use of error-prone DNA polymerases and reaction conditions. Here, Taq DNA polymerase was employed for the performance of error-prone PCR, as Taq DNA polymerase lacks proofreading acitvity and is therefore inherently error-prone. The error rate of Taq DNA polymerase could be further increased by employing a PCR reaction buffer containing $Mn^{2+}$. Further details concerning the reaction conditions of error-prone PCR are disclosed in Griesbeck et al., J. Biol. Chem., 276, 29188-29194 (2001) which is hereby incorporated by reference.

Subsequently, randomized DNA sequences produced by error-prone PCR were cloned into expression vectors and competent E. coli JM109(DE3) bacteria were transformed with these expression vectors and plated on LB/agar at 37 °C. The resulting mutant library clones were screened for clones expressing Ds-RED mutants of interest that still showed red fluorescence or even improved fluorescence and/or maturation properties.

To determine the oligomeric state of the Ds-RED mutants of interest. a single E. coli colony expressing the DS-RED mutant of interest was restreaked on LB/agar and was allowed to mature at room temperature. After two days to two weeks the bacteria were scraped from the plate, extracted with *B-per II* and analyzed (not boiled, in order to maintain the oligomeric structure) by SDS/PAGE (BioRad), and the gel was imaged with a digital camera. Further details concerning the applied screening and mutagenesis are disclosed in Campbell et al., PNAS, 99, No. 12, 7877-7882 (2002) which is hereby incorporated by reference.

The random mutagenesis with error-prone PCR and the subsequent screening of this library of mutated Ds-RED proteins as previously described finally resulted in the isolation of a monomeric derivate of Ds-RED, called mRFP1 as set force in SEQ ID No. 2, that comprises a total of 33 point mutations with respect to the native Ds-RED as set force in SEQ ID No. 1, wherein the 33 point mutations are the following:

a) N42Q, V44A, K163M, V175A, F177V, S197I, T217A, V71A (hotspots 1 - 3, mutations internal of the β-barrel)
b) K83L, F124L, L150M, S179T, V195T (mutations internal of the β-barrel)
c) I125R, V127T, I180T (mutations at the AB interface)
d) R153E, H162K, A164R, L174D, Y192A, Y194K, H222S, L223T, F224G, L225A (mutations at the AC interface)
e) R2A, K5E, N6D (mutations which reduce aggregations)
f) T21 S, H41 T and C117E (additional beneficial mutations).

The mutations of group a) had proved to be the most essential mutations clustering into three "hotspots".

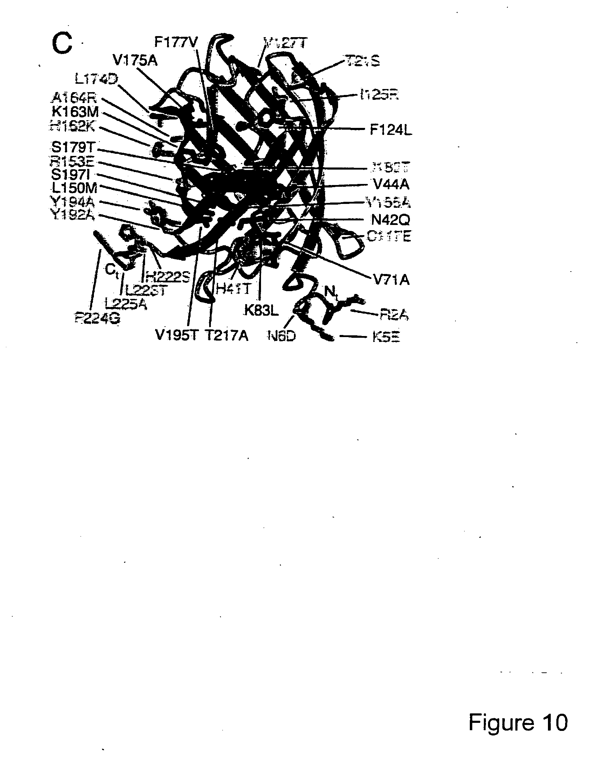

The first of these hotspots is found in the plane of the chromophore (when orientated as in Fig. 10) and is defined by positions N42 and V44 and the Q66 side chain of the chromophore. The mutations N42Q and V44A originated in the DS-Red mutant T1 where they bestowed the tetramer with a greatly improved maturation time, possibly by positioning Q66 in a conformation that promotes the oxidation which is responsible for the transformation of the immature green intermediate into the mature red chromophore.

The second hotspot is just above the plane of the chromophore (see Fig. 10), centered on the side chain K163 and influenced by V175 and F177, and possibly by I161. In the crystal structure of Ds-RED, the phenolate anion of the chromophore is stabilized by a salt bridge with the primary amine of K163. As experiments have shown, neither the K163Q mutation nor the K163M mutation in mRFP1 can participate in a salt bridge and therefore the polarization of the chromophore may be significantly different in these variants.

The third and most sensitive hotspot is just found below the plane of the chromophore and is centered on the side chains of K70 and the adjacent S197 and T217 (see Figure 10). Conservative mutations such as K70R, S197T and T217S had dramatic effects on the fluorescent properties of Ds-RED mutants and were critical to intermediate steps toward mRFP1. Therefore the mutation at the positions N42, V44, K163, V 175, F177, S197, T217, V71 of the native Ds-RED protein and in particular the concrete mutations N42Q, V44A, K163M, V175A, F177V, S197I, T217A, V71A seem to be crucial for the generation of a monomeric, red fluorescent derivate of Ds-RED as set force in SEQ ID No. 1 with excellent fluorescence and maturations properties for being used in combination with the green fluorescent protein for dcFCCS.

Thus, in this preferred embodiment of the screening process one the the autofluorescent proteins is a green autofluorescent protein and the other autofluorescent protein is a red autofluorescent protein which is preferably a dimeric - or more preferably a monomeric - derivate of Ds-RED as set force in SEQ ID No. 1.

Hereby, the monomeric derivate of Ds-RED as set force in SEQ ID No. 1 comprises point mutations that are internal to the β-barrel of Ds-RED and that are therefore crucial for protecting the fluorophore of Ds-Red from the environment. The monomeric Ds-RED derivate as set force in SEQ ID No. 1 comprises the non-silent mutations that are internal to the β-barrel of Ds-RED and that are located at the critical positions N42, V44, K163, V175, F177, S197, T217, V71 and/or at the corresponding amino acid positions which directly flank the aforementioned critical positions: Preferably, the monomeric Ds-RED derivate additionally comprises the non-silent mutations which are also internal to the β-barrel at the critical positions K83, F124, L150, 5179, V195.

The non-silent point mutations of the aforementioned positions are preferably non-conservative mutations. "Conservative mutations" are replacements of single amino acids with a residue having a specific property (i.e. a residue containing a positive charge, being polar, being lipophilic) by another amino acid with a residue having changed specific properties (i.e. a residue containing a negative charge, being unpolar, being hydrophilic).

In another preferred embodiment of the screening process, the non-silent point mutations are N42Q, V44A, K163M, V175A, F177V, S1971, T217A, V71A and/or K83L, F124L, L150M, S179T, V195T.

Furthermore, the monomeric derivate of Ds-RED as set force in SEQ ID No. 1 preferably comprises additional non-silent point mutations which destroy the interface between the A and B monomers in the native Ds-RED tetramer. More preferably, these non-silent mutations destroying the AB interface region are located at the critical positions I125, V127, I180 and/or at the corresponding amino acid positions which directly flank the aforementioned critical positions. The non-silent point mutations at the aforementioned positions are preferably non-conservative mutations. Particularly, the non-silent point mutations are I125R, V127T, I180T.

Furthermore, the monomeric derivate of Ds-RED as set force in SEQ ID No. 1 preferably comprises additional non-silent point mutations which destroy the interface between the A and C monomers in the native Ds-RED tetramer. More preferably, these non-silent mutations destroying the AC interface region are located at the critical positions R153, H162, A164, L174, Y192, Y194, H222, L223, F224, L225 and/or at the corresponding amino acid positions which directly flank the aforementioned critical positions. The non-silent point mutations at the aforementioned positions are preferably non-conservative mutations. Particularly, the non-silent point mutations are R153E, H162K, A164R, L174D, Y192A, Y194K, H222S, L223T, F224G, L225A.

In another preferred embodiment of the in vivo screening process, the monomeric derivate of Ds-RED as set force in SEQ ID No. 1 comprises non-silent mutations at the positions N42, V44, K163, V175, F177, S197, T217, at the positions 1125, V127, 1180, at the positions R153, H162, A164, L174, Y192, Y194, H222, L223, F224, L225 and/or at the corresponding amino acid positions which directly flank these positions.

Furthermore, the monomeric derivate of Ds-RED as set force in SEQ ID No. 1 preferably comprises additional non-silent point mutations which reduce aggregations. More preferably, these non-silent mutations reducing aggregations are located at the critical positions R2, K5, N6, and/or at the corresponding amino acid positions which directly flank the aforementioned critical positions. The non-silent point mutations at the aforementioned positions are preferably non-conservative mutations. Particularly, the non-silent point mutations are R2A, K5E, N6D. In another preferred embodiment of the screening process, the monomeric derivate of Ds-RED as set force in SEQ ID No. 1 comprises non-silent mutations at the positions N42, V44, K163, V175, F177, S197, T217 at the positions 1125, V127, 1180, at the positions R153, H162, A164, L174, Y192, Y194, H222, L223, F224, L225, at the positions R2, K5, N6 and/or at the corresponding amino acid positions which directly flank these positions.

Furthermore, the monomeric derivate of Ds-RED as set force in SEQ ID No. 1 preferably comprises the additional non-silent beneficial point mutations at the positions T21, H41 and C117 and/or at the corresponding amino acid positions which directly flank the aforementioned positions. The non-silent point mutations at the aforementioned positions are preferably non-conservative mutations. Particularly, the additional non-silent beneficial point mutations are T21S, H41T and C117E. In another preferred embodiment of the screening process, the monomeric derivate of Ds-RED as set force in SEQ ID No. 1 comprises non-silent mutations at the positions N42, V44, K163, V175, F177, S197, T217, at the positions I125, V127, I180, at the positions R153, H162, A164, L174, Y192, Y194, H222, L223, F224, L225, at the positions R2, K5, N6, at the positions T21, H41, C117 and/or at the corresponding amino acid positions which directly flank these positions.

In an other preferred embodiment of the screening process, the monomeric derivate of Ds-RED as set force in SEQ ID No.1 comprises the following point mutations: N42Q, V44A, K163M, V175A, F177V, S197I, T217A, V71A and/or K83L, F124L, L150M, S 179T, V195T (mutations internal of the β-barrel), I125R, V 127T, I180T (mutations at the AB interface), R153E, H162K, A164R, L174D, Y192A, Y194K, H222S, L223T, F224G, L225A (mutations at the AC interface), R2A, K5E, N6D (mutations which reduce aggregations), T21S, H41T and C117E (additional beneficial mutations).

In the most preferred embodiment of the screening process the monomeric derivate of Ds-RED as set force in SEQ ID No.1 is the monomeric derivate of Ds-RED as set force in SEQ ID No. 2 with the proposed non-silent point mutations at all mentioned mutation sites (see SEQ ID No. 2, see Figure 10).

In another preferred embodiment of the screening process, in step a) a first fusion protein comprising a green autofluorescent protein and a second fusion protein comprising the a autofluorescent protein, preferably mRFP1 (see SEQ ID No. 2), are expressed in a cell and in step b) the coincidently occurring fluorescence signals of the green and the red autofluorescent proteins in the cells of step a) are determined by comparing the measured fluorescence cross correlation signal with the corresponding cross correlation signal measured for cells, which had been transformed with a negative control vector, indicating 0 % protein-protein-interaction, and by comparing the measured fluorescence cross correlation signal with the corresponding cross correlation signal measured for cells, which had been transformed with a positive control vector, indicating 100 % protein-protein-interaction.

The negative control vector, indicating 0 % protein-protein-interaction, is preferably an expression vector comprising the negative control construct which contains first the DNA coding for the applied green fluorescent protein, followed by an IRES-element, followed by the DNA coding for the applied red fluorescent protein under the control of a single promoter. Naturally, the negative control construct might also contain the elements in the opposite order, that means first the DNA coding for the red green fluorescent protein, followed by an IRES-element, followed by the DNA coding for the applied green fluorescent protein under the control of a single promoter. Preferably the expression vector that is used as negative control vector is the same expression vector that is used for expressing the first and/or the second fusion protein. Since usually the applied red and the applied green fluorescent proteins do not interact with one another at all, the cross correlation measured for the applied red and green fluorescent proteins which are not coupled to each other represents the "base line" of cross correlation which will also be measured when no protein-protein interaction occurs and this basic cross correlation has to be subtracted from the cross correlation measured in the experiment.

If the green fluorescent protein applied in the screening process is EGFP and the red fluorescent protein applied in the screening process is mRFP1, then the negative control vector preferably comprises the construct EGFP-IRES-mRFP1 or mRFP1-IRES-EGFP.

The positive control vector indicating 100 % protein-protein-interaction is preferably an expression vector comprising the DNA coding for a fusion protein consisting of the applied green fluorescent protein fused in frame to the applied red fluorescent protein or vice versa under the control of a promoter. Hereby, the expression vector that is used for the positive control vector is preferably the same vector that is used for expressing the first and/or the second fusion protein. If the green fluorescent protein applied in the screening process is EGFP and the red fluorescent protein applied in the screening process is mRFP1, than the positive control vector preferably comprises comprises the DNA coding for a fusion protein consisting of EGFP fused in frame to the mRFP1 (SEQ ID No. 2) or vice versa under the control of a promoter. Since in the expressed EGFP/mRFP 1 or mRFP1/EGFP fusion protein the two fluorescent proteins EGFP and mRFP1 obviously are in the spatially closest proximity that should be possible, the emission signals in the red channel and in the green channel should be close to 100 % coincident. Thus, the highest cross correlation signal that is possible should be measured for this positive control construct. In so far 100 % protein-protein-interaction, that means a positive assay result, should show the same (or at least similar) cross correlation signal as this positive control construct.

Another object of the invention is an assay kit containing a first expression vector comprising the DNA coding for the mRFP1 protein fused to a multiple cloning site under control of a promoter and containing a second expression vector comprising the DNA coding for the EGFP protein or for another variant of the GFP-family fused to a multiple cloning site under control of a promoter. It is preferred that the DNA coding for the individual members of a protein library has been cloned into the multiple cloning site of either the first or of the second expression vector. Preferably the promoters of the first and of the second expression vector are the same promoters. It is further preferred that the assay kit contains only a one expression vector which comprises a promoter followed by the DNA coding for the mRFP 1 protein fused to a multiple cloning site, followed by an IRES-element, followed by the DNA coding for the EGFP protein or for another variant of the GFP-family fused to a multiple cloning site. Hereby the DNA coding for the two fusion proteins can also be cloned in the opposite order. It is also preferred that the assay kit contains only a one expression vector which comprises a bidirectional promoter with the DNA coding for the mRFP1 protein fused to a multiple cloning site being cloned in 5'-direction of the bidirectional promoter and with the DNA coding for the EGFP protein or for another variant of the GFP-family fused to a multiple cloning site being cloned in 3'-diretion of the bidirectional promoter or vice versa.

Another object of the invention is the use of two individual autofluorescent proteins, which differ from each another in at least one detectable photophysical parameter, for detecting protein-protein interactions by fluorescence cross correlation spectroscopy (FCCS) in vivo, wherein the fluorescence cross correlation signal represents the coincidently occurring fluorescence signals of the first and the second autofluorescent proteins. The at least one photophysical parameter which differs between the two individual autofluorescent proteins are either different emission wavelengthes of the two autofluorescent proteins, different fluorescence life times of the two autofluorescent proteins, or different polarisations of the fluorescent light emitted by each of the two auto fluorescent proteins.

Hereby the use of two individual autofluorescent proteins, which differ from each another in their emission wavelengthes, for detecting protein-protein interactions by fluorescence cross correlation spectroscopy (FCCS) in vivo is preferred. Further preferred is the use of a first autofluorescent protein showing a green autofluorescence and of a second autofluorescent protein showing a red fluorescence for detecting protein-protein interactions by fluorescence cross correlation spectroscopy (FCCS) in vivo. Hereby, the second autofluorescent protein showing a red fluorescence is preferably a dimer or monomer, more preferably a dimeric or monomeric derivate of Ds-RED as set force in SEQ ID No. 1. The monomeric derivate of Ds-RED as set force in SEQ ID No. 1 preferably comprises the aforementioned non-silent mutations and is in particular the red fluorescent mRFP 1 as set force in SEQ ID No. 2.

In order to demonstrate that the red fluorescent mRFP1 and the green fluorescent EGFP are suitable for detecting protein-protein interactions by measuring dcFCCS in vivo, Fos and Jun, the two protein components of the AP-1 transcription factor, which are known to exert their function as a heterodimer, were used to clone two expression vector constructs, one containing the the Fos-EGFP fusion and the other containing the Jun-mRFP1 fusion. The eukaryotic expression vector containing the Fos-EGFP fusion was permanently integrated into a HeLa cell line, then the clone was transiently super-transfected with an eukaryotic expression vector containing the Jun-mRFP1 fusion. Since Fos and Jun, each linked to EGFP and mRFP respectively, are known to interact in vivo, this protein-protein interaction should be detectable by measuring a strong fluorescence cross correlation signal in the living cells.

The following figures describe the objects of the invention, in particular the suitability of red fluorescent proteins for detecting protein-protein-interactions by measuring FCCS in vivo, in more detail.

**Figure 1**: Normalised autocorrelation amplitudes $G(\tau)^*N$ in the red (filled circles) and the green channel (open circles) measured in cells expressing separately EGFP and niRFP1. The autocorrelation amplitude is displayed against the logarithm of the correlation time $\tau$. The cross correlation function is depicted in filled squares.
Insert: confocal images of cells in the EGFP (A) and the mRFP 1 channel (B).

**Figure 2**: Normalised autocorrelation amplitudes $G(\tau)^*N$ in the red (filled circles) and the green channel (open circles) measured in HeLa cells expressing a fusion of EGFP and mRFP1. The cross correlation function is depicted in filled squares.
Insert: confocal images of cells in the EGFP (A) and the mRFP 1 channel (B).

**Figure 3**: Normalised autocorrelation amplitudes $G(\tau)^*N$ in the red (filled circles) and the green channel (open circles) measured in cells expressing the AP1 deletion mutants c-FosΔDNAΔdim-EGFP and c-JunΔDNAΔ dim-mRFP1. The cross correlation function is depicted in filled squares.
Insert: confocal images of cells in the EGFP (A) and the mRFP 1 channel (B).

**Figure 4:** Normalised autocorrelation amplitudes $G(\tau)^*N$ in the red (filled circles) and the green channel (open circles) measured in cells expressing c-Fos-EGFP and c-Jun-mRFP1. The cross correlation function is depicted in filled squares.
Insert: confocal images of cells in the EGFP (A) and the mRFP 1 channel (B).

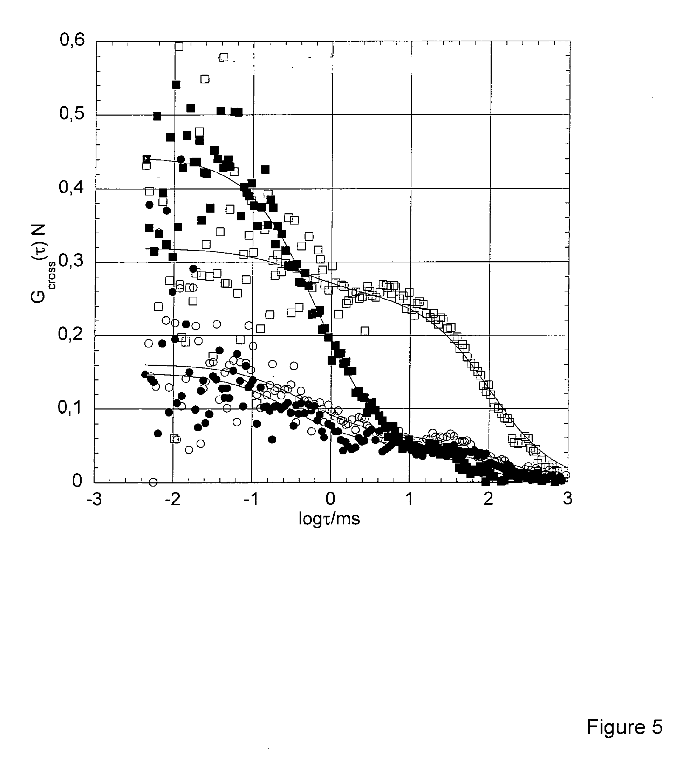

**Figure 5:** Summary of normalised cross correlation amplitudes $G_{cross}(\tau)^*N$ measured in cells expressing EGFP and mRFP1 (filled circles), the deletion mutants c-FosΔDNAΔdim-EGFP and c-JunΔDNAΔdim-mRFP1 (open circles), the fusion protein EGFP-mRFP 1 (filled squares) and the fusion proteins c-Fos-EGFP and c-Jun-mRFP 1 (open squares).

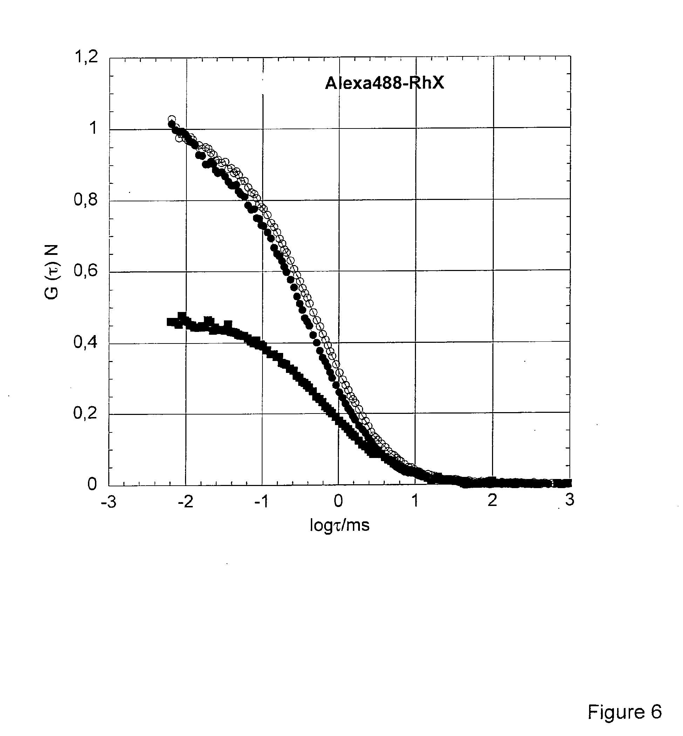

**Figure 6:** Normalised autocorrelation amplitudes $G(\tau)^*N$ in the red (filled circles) and the green channel (open circles) measured for the double-labelled, double-stranded 157 bp DNA fragment which was labelled on the one end with rhodamine X and on the other end with an Alexa 488 dye. The cross correlation function is depicted in filled squares.

Insert: scheme of the double-labelled DNA fragment.

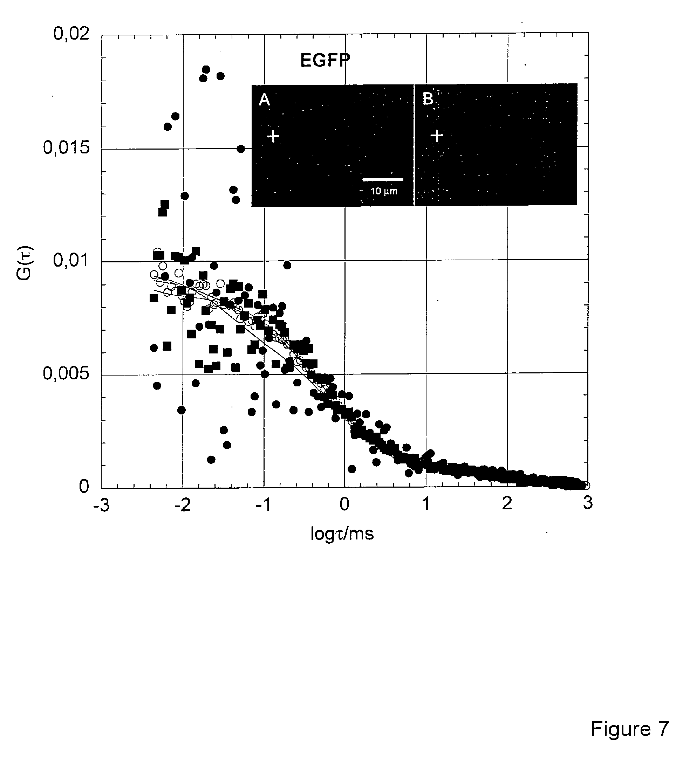

**Figure 7:** Autocorrelation amplitudes G(τ)*N in the red (filled circles) and the green channel (open circles) measured in cells expressing EGFP. The cross correlation function is depicted in filled squares.
Insert: confocal images of cells in the EGFP (A) and the MRFP1 channel (B).

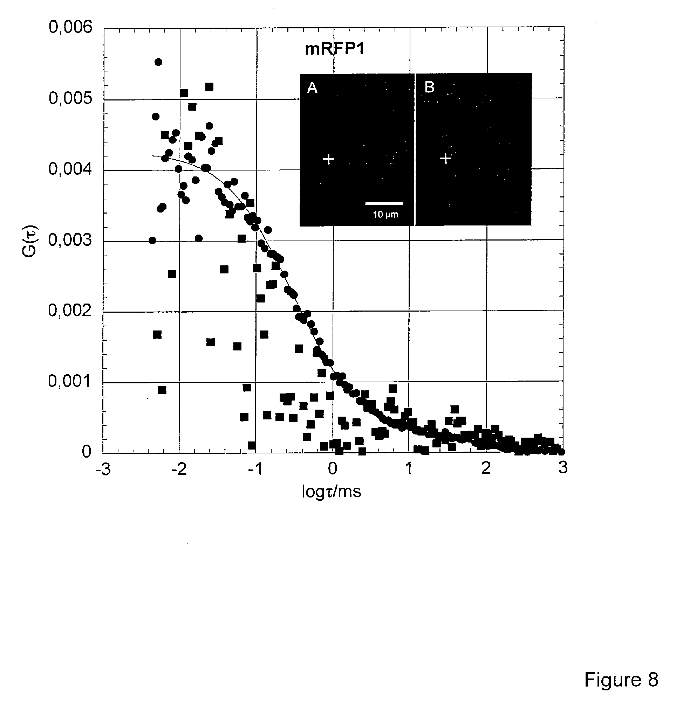

**Figure 8:** Autocorrelation amplitudes G(τ)*N in the red (filled circles) and the green channel (open circles) in cells expressing mRFP (no autocorrelation amplitude in the green channel).. The cross correlation function is depicted in filled squares.
Insert: confocal images of cells in the EGFP (A) and the mRFP channel (B).

**Figure 9:** Normalised autocorrelation amplitudes G(τ)*N in the red (filled circles) and the green channel (open circles) measured in cells expressing c-Fos-EGFP and c-Jun-eqFP611. The cross correlation function is depicted in filled squares.

**Figure 10**: Graphical representation of mRFP1 (see SEQ ID No. 2) with its mutations in comparison to native Ds-RED (see SEQ ID No. 1) based on the X-ray crystal structure of DsRED. The darker printed mutations are those mutations which are internal to the β-barrel, whereas the lighter printed mutations represent external residues.

The following examples describe the objects of the invention in more detail.

### 1. Example: Plasmid constructions

#### 1.1. AP1 fusion proteins

As a starting vector for cloning purposes the pSV-EYFP vector was used. The pSV-EYFP had been derived from the pECFP-1 vector (Clontech) by inserting the SV40 promoter into the HindIII restriction site in the multiple cloning site (MCS) and by exchanging the blue fluorescent protein ECFP against the yellow fluorescent protein EYFP.
For generating the vector pSV-c-Fos-EGFP the coding sequence of the human c-Fos protein (donated by P. Angel) was amplified by PCR using the following forward and reverse primers: 5'-CTT CGA ATT CTG ATG ATG TTC TCG GGC TTC AAC GC-3' and 5'-CGG TGG ATC CCG CAG GGC CAG CAG CGT GGG TGA GC-3', then the amplified fragment and the vector pSV-EYFP were digested with the restriction enzymes EcoRI and BamHI and finally ligated. In a second cloning step, EYFP was exchanged for EGFP by using the AgeI and NotI restriction sites, resulting in the final vector pSV-c-Fos-EGFP.
The vector pSV-c-Jun-mRFP 1 was cloned in a multi step strategy. The first part of the coding region of the human c-Jun from pBAT-c-Jun (provided by P-Arigel) was obtained by restriction of pBAT-c-Jun with HindIII and BamHI and inserted into the MCS of pSV-EYFP. The second part of c-Jun was amplified by PCR using the following primers: 5'-CGG CTG CAG GCC CTG AAG GAG GAG CCT CAG ACA GT-3' and 5'-CGG TGG ATC CCG AAA TGT TTG CAA CTG CTG CG-3'. The amplified fragment and the vector p-c-JunI-EYFP (without SV40 promoter) were digested with PstI and BamHI and then ligated. Subsequently the SV40 promoter was inserted again into the HindIII restriction site resulting in the vector p-c-Jun-EYFP. In the last step the coding sequence for the monomeric Red Fluorescent Protein 1 (mRFP1) was amplified from pRSET$_B$-mRFP1 (donated by R. Tsien) with the following primers: 5'-AAT TTA CCA CCG GTC ATG GCC TCC TCC GAG GAC G-3' and 5'-TAA ATT GCG GCC GCT TTA GGC GCC GGT GGA GTG GCG-3'. In order to exchange EYFP by mRFP the amplified mRFP1-fragment and the vector p-c-Jun-EYFP were finally digested with the restriction enzymes AgeI and NotI and ligated, resulting in the final vector pSV-c-Jun-mRFP1.

#### 1.2. AP 1 deletion mutants

##### FosΔdimΔDNA-EGFP

The DNA binding domain (417 bp - 480 bp) and the dimerisation domain (495 bp - 579 bp) of c-Fos were removed by PCR amplification of c-Fos with following primers: 5'-CTT CGA ATT CTG ATG ATG TTC TCG GGC TTC AAC GC-3' and 5'-ATA AAT TGG ATC CCG CTC TTC TTC TTC TGG AGA TAA CTG TTC C-3'. The amplified fragment was digested with EcoRI and BamHI and inserted in the MCS of pSV-EGFP.

##### JunΔdimΔDNA-mRFP1

For the construction of the deletion mutant of c-Jun the vector RSV-c-JunΔ146-220 (P. Angel, DKFZ) was digested with HindIII and BglII obtaining the first 435 bp of the coding region of c-Jun and inserted into the HindIII and BamHI sites of pSV-EYFP. Afterwards the SV 40 promoter region was inserted in the HindIII restriction site. In the last step the mRFP 1 was integrated in the vector like above described (see description for the generation of pSV-c-Jun-mRFP1).

Control plasmid pSV-EGFP-mRFP1

The coding sequence of the Enhanced Green Fluorescent Protein (EGFP) from pEGFP-N1 (Clontech, Palo Alto, CA, USA) was amplified by PCR using the forward and reverse primers: 5'-AAT TAA CAG TCG ACG ATG GTG AGC AAG GGC GAG G-3' and 5'-AAT ATA TGG ATC CCG CTT GTA CAG CTC GTC CAT GC-3'.
The amplified fragment and the plasmid pSV-mRFP1 (constructed by G. Müller and N. Baudendistel) were digested using SalI and BamHI restriction enzymes and then the amplified fragment was ligated with the pSV-mRFP1 vector to construct the vector pSV-EGFP-mRFP1 comprising the DNA coding for the fusion protein pEGFP-mRFP1. The linker between EGFP and mRFP1 hereby contains 7 amino acids (RDPPVAT) originating from base pairs of the multiple cloning site.

Control plasmid pIRES2-EGFP-mRFP1

The coding sequence of the monomeric Red Fluorescent Protein (mRFP1) was amplified from $pRSET_B$-mRFP1 by PCR using the forward and reverse primers: 5'-AAT TAA CAG TCG ACG ATG GCC TCC TCC GAG GAC G-3' and 5'-AAT TTA TGG ATC CCG TTA GGC GCC GGT GGA GTG GCG-3' for mRFP1.
The amplified fragment and the plasmid pIRES2-EGFP (Clontech) were digested using SalI and BamHI and then ligated, generating the vector pIRES2-EGFP-mRFP1.

Double-stranded 157 bp DNA-fragment double-labelled with Alexa 488 and rhodamine X

For the construction of the 157 bp DNA-fragment, labelled with Alexa 488 and rhodamine X, the 5S positioning sequence of Xenopus Borealis pXP 10 was amplified by PCR using the labelled forward and reverse primers: 5'-Alexa488-GAA TTC GAG CTC GCC CGG GGA-3' and 5'-RhoX-GTA CTA ACC AGG CCC GAC-3' purchased by Thermo (Ulm, Germany).

2. Example: Cell culture

The transfections of the mammalian expression vectors were carried out with FuGene 6 from Roche Diagnostics (Mannheim, Germany) as proposed by the manufacturer.
Adherent HeLa cells were grown in a 5% $CO_2$ humidified atmosphere at 37 °C and passaged in RPMI 1640 without phenol red (purchased from Invitrogen Life Technologies, Carlsbad, CA, USA) supplemented with 10% fetal calf serum (purchased from Invitrogen Life Technologies).
For *in vivo* imaging, cells were cultured sub-confluent, transfected in falcons and then transferred to chambered cover slips from Nunc (Wiesbaden, Germany). For the measurement of auto- and cross correlations the cover slips were mounted on the scanning stage of the FCCS unit in an incubator chamber at 37 °C.

3. Example: description of the dcFCCS-setup and calibration

The interaction between the two fusion proteins Fos-EGFP and Jun-mRFP1 was measured by using an FCCS setup consisting of a confocal excitation/detection module and a galvanometer mirror scanner as described *in Langowski, J. & Tewes, M in Protein-DNA Interactions: A Practical Approach (eds. Travers, A. & Buckle, M.) 95-111 (Oxford University Press, Oxford, 2000)* and in *Wachsmuth, M. et al. Analyzing intracellular binding and diffusion with continuous fluorescence photobleaching. Biophys J 84, 3353-63. (2003).*
The instrument was calibrated using 5-(6-)carboxy-fluorescein (Molecular Probes, Eugene, Oregon, USA) for the green channel and Alexa 568 carboxylic acid, succinimidyl ester (Molecular Probes) for the red channel as standards for the diffusion times and focal volume size.
The data were fitted with a model (Eq. 2 + 3, supplementary methods section) containing one diffusional and one non-fluorescent component representing the triplet state of the dyes.
The diffusion time of Alexa 568 (58.5 $\mu$s $\pm$ 1.1 $\mu$s) was 20% slower than the diffusion time of 5-(6-)carboxy-fluorescein (48.4 $\mu$s $\pm$ 3.6 $\mu$s, D = 2.6 $10^{-10}$ m²/s) reflecting the greater detection volume seen by the red channel due to the dependence of the observation volume on the excitation wavelength.
In order to calibrate the overlap of the detection and excitation volume and as a standard for maximum cross-correlation (100%) a 157 bp long, double-stranded DNA oligonucleotide was used, which was labelled on the one end with a rhodamine X dye and on the other end with an Alexa 488 dye. The autocorrelation curves hereby obtained in the green and the red channel were normalised, since the amplitudes $\tilde{G_x}(\tau)$ and $\tilde{G_y}(\tau)$ were identical, corresponding to equal concentrations of Alexa 488 and rhodamine X. Also the cross-correlation function between the red and the green channel was calculated and compared to the corresponding autocorrelation curves. The maximum cross-correlation amplitude for the 100 % double-labelled probe at the concentration of 250 mM amounts to 46% $\pm$ 1% (n=5).

4. Example: The FCCS measurements of positive controls (=100 % interaction) and of negative controls (0 % interaction)

The diffusion properties of free EGFP and free mRFP1, both proteins co-expressed at equal levels, and a fusion construct consisting of EGFP and mRFP1 in living HeLa cells were analysed.

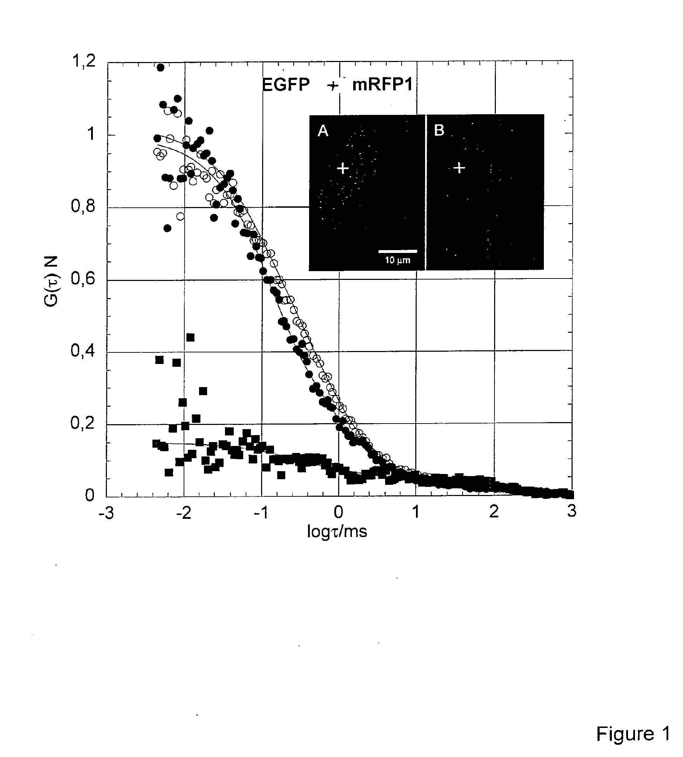

When EGFP and mRFP1 were expressed separately at equal levels by transfecting the pIRES-EGFP-mRFP1 vector into HeLa cells, the cross-correlation amplitude was only 13% $\pm$ 3% (n=16) of the autocorrelation signal (see Fig. 1).

As a negative control for FCCS, auto- and cross-correlation curves were recorded in cells transfected with the pIRES-EGFP-mRFP1 vector. The pIRES vector is a bicistronic vector (Clontech), which produces one messenger RNA carrying the genetic information of both proteins (EGFP and mRFP1), which is translated into two separate proteins. In this case nearly the same amounts of mRFP1 and EGFP were produced (in some cases a slightly greater amount of mRFP1 was produced, but such cells were not used).

For comprehensibility the auto and cross-correlation curves were normalised by the amplitude (for the auto correlation the respective amplitude of each channel was taken for normalisation, for the cross-correlation the average amplitude obtained in the green and the red channel was chosen) (see Fig. 1). However, the cross-correlation amplitude only provides a very noisy signal, indicating that the fluorescence fluctuations are not correlated having two separate proteins. The cross-correlation amplitude is calculated to 13% $\pm$ 3% (n=16).

The diffusion time amounts to 0.49 ms $\pm$ 0.09 ms in the green channel and to 0.42 ms $\pm$ 0.12 ms in the red channel. This measurement is in good agreement with the results of the control experiments with free EGFP alone or free mRFP1 alone in cells.

Thus, the measured cross correlation of **13% $\pm$ 3% (n=16)** was used as background correlation due to spectral crosstalk between the two colour channels and served as the reference for no interaction at all (0% interaction, **negative control).**

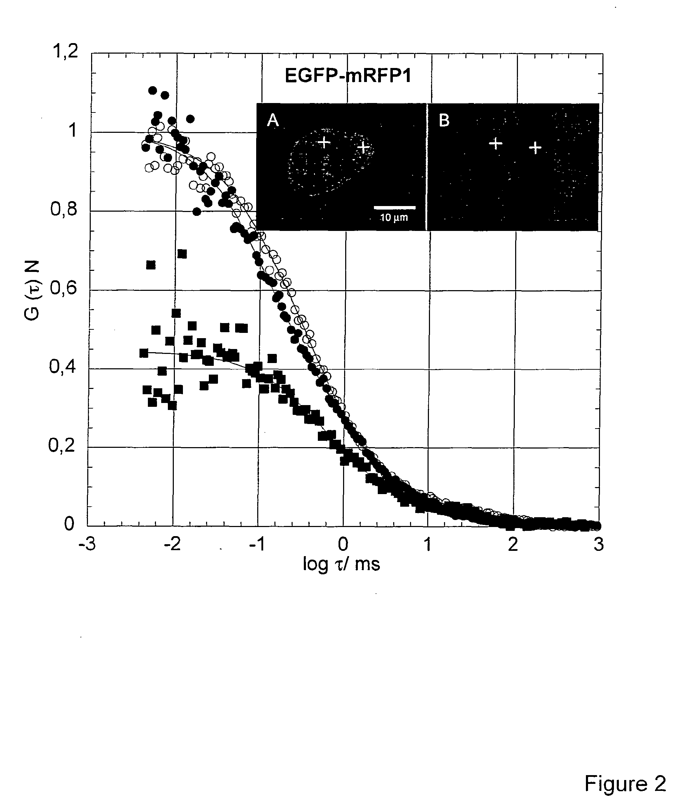

On the other hand, cells expressing the two-colour fusion protein comprising EGFP and mRFP1 showed a strong cross-correlation signal with 45% $\pm$ 4% (n=14) of the amplitude of the autocorrelation signal in either colour channel (see Fig. 2), which corresponds very well to the cross correlation value of 46 % as measured free in solution for the double-labelled double-stranded oligonucleotide control. This amplitude was therefore taken as the reference level for complete dimerisation (100% interaction). In Fig. 2 the normalised auto- and cross-correlation curves of the EGFP-mRFP 1 fusion protein are shown. Here a slower diffusion time in both channels of 0,66 ms $\pm$ 0,25 ms is obtained showing a bigger complex and/or a changed secondary structure of the fusion protein compared with the single proteins.

Thus, the measured cross correlation of **45% $\pm$ 4% (n=14)** was used as a **positive control** representing 100% protein-protein interaction.

Autofluorescence intensities in wild type HeLa cells (non-transfected HeLa cells) at the same laser excitation intensities of about 10 kW/cm$^2$ were smaller than 1% and therefore negligible.

5. Example: fluorescence cross correlation for Fos-EGFP and Jun-mRFP1 and for Fos$\Delta$dim$\Delta$DNA-EGFP and Jun$\Delta$dim$\Delta$DNA-mRFP 1

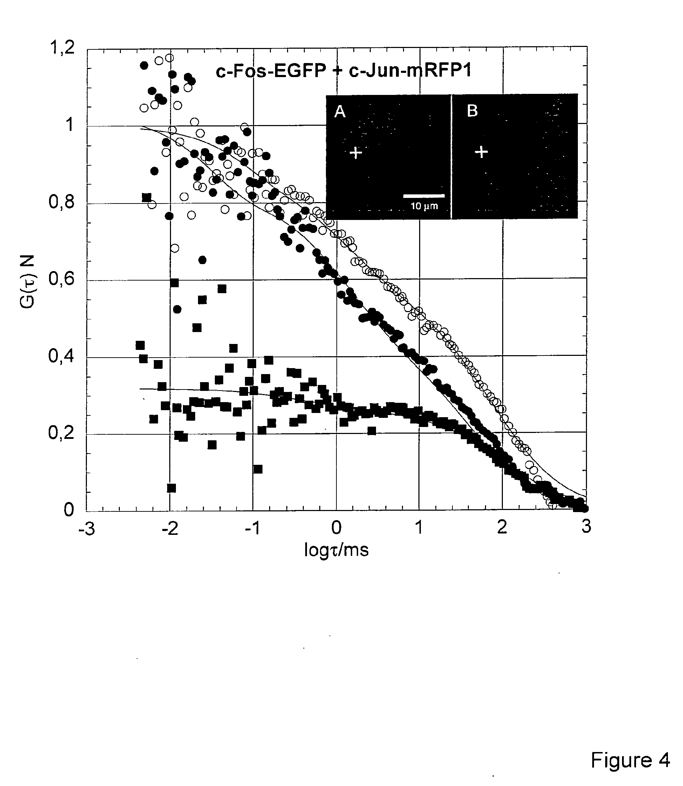

The fluorescence cross correlation signal was measured for living cells which expressed equal levels of Fos-EGFP and Jun-mRFP1 fusion proteins. To measure the interactions of c-Fos and c-Jun, a HeLa cell line which first had been stably transfected with an expression vector comprising the c-Fos-EGFP fusion, was super-transfected with the an expression vector comprising the c-Jun-mRFP1 fusion. The cell picture in Fig. 4 shows the fluorescence in the green and the red channel as well as the auto- and cross correlations for these cells. The main fluorescence in both channels is localised in the nucleus due to the nuclear targeting signal (NLS) in both proteins, c-Fos and c-Jun.

Furthermore, the fluorescence cross correlation signal was also measured for living cells which expressed equal levels of the deletion mutants Fos$\Delta$dim$\Delta$DNA-EGFP and Jun$\Delta$dim$\Delta$DNA-mRFP1, wherein both the dimerisation domain ($\Delta$dim) and the DNA-binding domain ($\Delta$DNA = basic Zipper domains) of the Fos and Jun proteins had been removed.

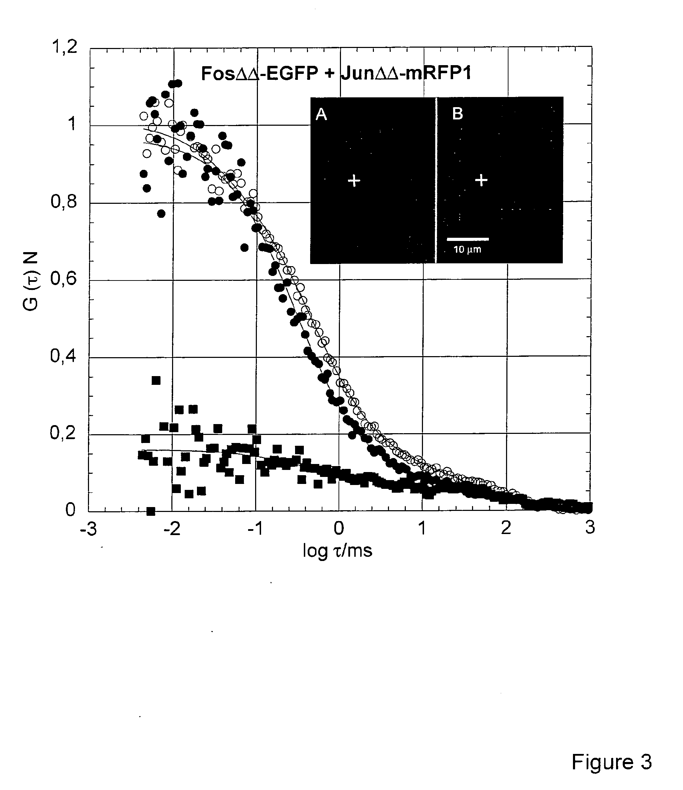

While the cross-correlation amplitude for the deletion constructs **(Fos$\Delta$dim$\Delta$DNA-EGFP and Jun$\Delta$dim$\Delta$D-NA-mRFP1)** was at the background level with **18% $\pm$ 4%** (n=12) (see Fig. 3), cells that expressed the full-length fusion proteins **(Fos-EGFP and Jun-mRFP1)** showed an increased cross-correlation amplitude of **31% $\pm$ 6%** (n=19) (see Fig.

4). A t-test showed this difference to be significant at a level of p < 0.01. Thus, FCCS unambiguously shows the interaction between Fos and Jun in the living HeLa cell. It can be concluded from that experiment that the red fluorescent protein mRFP1 as set force in SEQ ID No. 2 is suitable to be used in combination with EGFP for an assay for detecting protein-protein interactions by measuring fluorescence cross correlation (FCCS) in vivo.

Due to the nuclear localisation site (NLS) in all AP1 proteins the majority of these proteins, except for those with the DNA binding domain deleted, are localised in the nucleus. In the cytoplasm the concentration of c-Fos and c-Jun is an order of magnitude smaller than in the nucleus. It was observed that in most cells the fluorescence in the cytoplasm was too small to record correlation curves, or the recorded correlation curves were too noisy to calculate exact cross-correlation amplitudes.

The cross-correlation function (see Fig. 4) of the Fos-Jun complex in the nucleus showed only a slowly decaying component. Free Fos-Jun dimers would be expected to exhibit a much faster diffusion time, similar to that of the EGFP-mRFP1 fusion proteins. (monomers and dimers cannot be distinguished by the diffusion time since $\tau_{diff} \propto M^{1/3}$, and this difference would be within the experimental uncertainty for the *in vivo* measurements). The fact that no fast diffusion component could be detected indicates that no free dimers are detectable in the cell: obviously the vast majority of Fos-Jun dimers are associated with DNA. Furthermore, it could be observed that a fusion construct of EGFP with Fos lacking only the dimerisation domain still showed nuclear localization and a slowly decaying component (40%) in the FCS autocorrelation function, indicating association of Fos monomers with large cellular components (data not shown). This interaction with large cellular components does not necessarily require the bZip (basic Zipper) domains of Fos and Jun proteins, as previously shown for protein-protein interaction with other transcription factors, such as GATA (McBride et al. *Oncogene* **22,** 8403-12 (2003)), FHL2 (Morlon et al., *Proc Natl Acad Sci U S A* **100,** 3977-82 (2003)) and Cbfal (Hess et al., *J Biol Chem* **276**, 20029-38 (2001)).

6. Example: cross correlation for c-Fos-EGFP and c-Jun-eqFP611

In Wiedenmann et al., PNAS, 99, No. 18, 11646-11651 (2002) the monomeric structure of the red fluorescent protein eqFP611 from sea anemone *Entacmaea quadricolor* was described. Therefore it seemed promising to try whether eqFP611 can also be used as red fluorescent protein in combination with the green fluorescent protein EGFP to detect protein-protein interactions between two hybrid proteins by measuring FCCS in living cells (in vivo two hybrid FCCS). For that purpose two expression vectors comprising the DNA fusion constructs c-Fos-EGFP and c-Jun-eqFP611 respectively were cloned. A HeLa cell line, which had been stably transformed with the expression vector comprising the fusion c-Fos-EGFP, was transiently transformed with the expression vector comprising the fusion c-Jun-eqFP611.

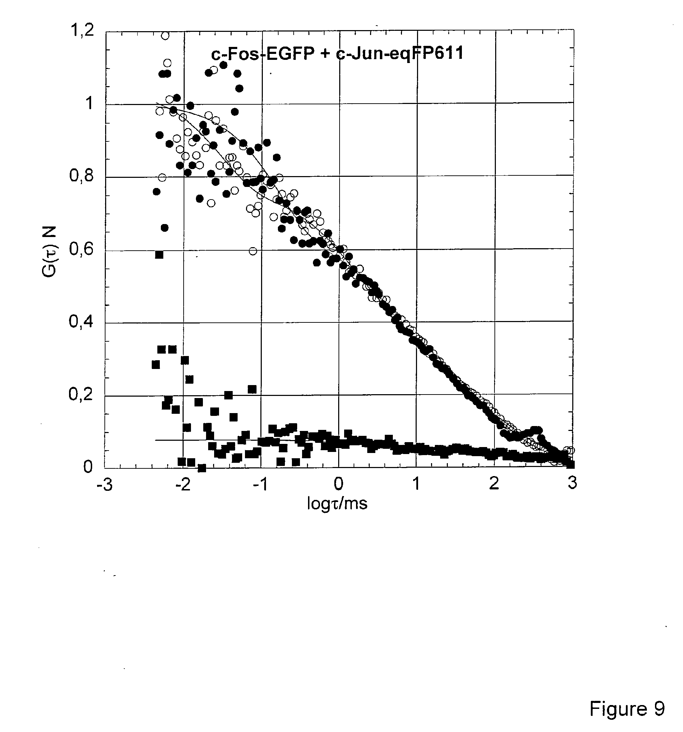

In Fig. 9 it is shown that no cross correlation signal could be obtained from HeLa cells expressing c-Fos-EGFP and c-Jun-eqFP611. The measured cross correlation curve shown in Fig. 9 is quite similar to that cross correlation curve measured for cells which express EGFP and mRFP1 separately (as shown in Fig. 1) which represents the cross correlation only due to spectral crosstalk between EGFP and mRFP 1. Thus, this experiment clearly shows that the occuring protein-protein-interaction between the Fos-part of c-Fos-EGFP and the Jun-part of c-Jun-eqFP611 obviously can not be detected by FCCS as it was clearly possible for the fusion protein pair c-Fos-EGFP and c-Jun-mRFP1. The main reason for the unsuitablity of eqFP611 for its use in combination with EGFP for performing two hybrid-FCCS in vivo is probably the fact that eqFP611 originally had been isolated from sea anemone *Entacmaea quadricolor* which has a temperature optimum of 30 °C instead of 37 °C. When the protein eqFP611 which folds properly at a temperature of 30 °C in *Entacmaea quadricolor* is expressed for instance in human cell lines at 37 °C, then the folding into the native three-dimensional structure might be impaired which consequently might negatively influence the physical properties of eqFP611.

7. Example: Experimental Setup and Data analysis

FCS and FCCS data analysis

The measured auto- and cross-correlation curves $G_x(\tau)$, $G_y(\tau)$ and $G_{xy}(\tau)$

$$G_{xy}(\tau) = \frac{\langle F_x(t)F_y(t+\tau)\rangle}{\langle F_x\rangle\langle F_y\rangle} - 1 \qquad\qquad \text{Eq.1}$$

were analyzed by fitting an appropriate model function (Eq.2) to the data, using the program *Quickfit* (written by Langowski et al., DKFZ, Germany) based on the Marquardt-Levenberg algorithm:

$$G_{xy}(\tau) = \frac{1}{c V_{eff,xy}} \left(1 + \frac{\tau}{\tau_{diff}}\right)^{-1} \left(1 + \frac{\tau}{\kappa^2 \tau_{diff}}\right)^{\frac{1}{2}} \qquad\qquad \text{Eq.2}$$

with

$$\tau_{diff} = \frac{\omega_0^2}{4D}$$

and

$$\kappa = \frac{z_0}{\omega_0},$$

whereby $\omega_0$ and $z_0$ are the lateral and axial dimensions of the focus.

For $x = y$ Eq. 1 defines the autocorrelation function $G_x(t)$ for one molecular species in a single detection channel, for $x \# y$ the equation provides the cross correlation function from two detection channels.

The fit with two fluorescent and one non-fluorescent component, representing the triplet state of the dyes according to Eq. 3, was performed:

$$G_{xy}(\tau) = \frac{1}{c V_{eff,xy}} \left(\frac{1 - \theta_1 + \theta_1 e^{-\tau/\tau_{imp}}}{1 - \theta_1}\right) \left(1 + \frac{\tau}{\tau_{diff}}\right)^{-1} \left(1 + \frac{\tau}{\kappa^2 \tau_{diff}}\right)^{\frac{1}{2}}. \qquad \text{Eq. 3}$$

For the FCCS measurements only cells with similar concentrations and with mean equal amplitudes of the red and the green protein were selected. All autocorrelation curves were normalized by the amplitude, obtained in the red or green channel, respectively. Cross-correlation curves were normalized by the average amplitude, obtained in the green and the red channel.

Fluorescence Fluctuation Microscopy (FFM)

Auto- and cross-correlation spectroscopy measurements were carried out on the Fluorescence Fluctuation Microscope (FFM) which was constructed as described in Weidemann et al., *Single Molecules* **3**, 49-61 (2002). This setup combines a FCS/FCCS module and a galvanometer mirror scanning unit attached to the video port of an inverted IX-70 microscope (Olympus, Hamburg, Germany) with a UplanApo 60X, 1.2 NA water immersion objective.

Intracellular measurements were performed in an incubator chamber mounted on the microscope (EMBL, Heidelberg, Germany), which maintains the temperature at 37 °C and provides a humidified 5% $CO_2$ atmosphere.

The EGFP fluorescence was excited with the 488 nm line of an Argon-Krypton laser from Omnichrome (Wessling, Germany) and the emission was detected from 515 nm to 545 nm with avalanche photodiodes (SPCM-AQR-13, Perkin-Elmer, Wellesley, MA, USA) after passing a 530 DF 30 band-pass filter.

The mRFP1 fluorescence was excited with the 568 nm line of the same laser and detected between 608 nm and 662 nm also with avalanche photodiodes after passing a 635DF 55 band-pass filter. For double excitation experiments a band-pass filter (490-577DBEX) and a dichroitic mirror (490-575DBDR) to split the light was used. All filters were purchased from Omega Optical (Brattleboro, VE, USA).

With a specific control software (written by Langowski et al, DKFZ, Germany) confocal images as well as white transmission light pictures could be acquired allowing he positioning of the laser with an accuracy of 30 nm for FCS- and FCCS-measurements to defined locations within the cells.

Signal fluctuations induced by molecules diffusing across the focus were recorded and directed into an ALV-5000/E correlator card (ALV Laser GmbH, Langen, Germany).

In summary, the described two-hybrid FCCS assay (=THFCCS)) represents a novel way of detecting protein-protein interactions *in vivo* using genetic tags. Other than previous methods based on FRET, it is not dependent on close spatial proximity of the fluorophores, but only on their correlated motion and on their coincident emissions. If the two hybrid FCCS is combined with an effective scanning system for the measurement of FCCS in living cells, this assay system will be suitable for screening protein-protein interactions in a large number of cells. Furthermore it would be possible to detect protein-protein interactions that only occur in specific subcellular locations (as for instance in the nucleus, see example 2). Thus, the automatic detection of protein-protein interactions in a high-throughput two hybrid-FCCS screen and the determination of the cellular compartment in which these interactions occur becomes feasible.

## Claims

1. Process for the detection of protein-protein-interactions in vivo comprising the following steps:

   a) expressing a first fusion protein comprising a first protein that is fused with a first autofluorescent protein and expressing a second fusion protein comprising a second protein that is fused with a second autofluorescent protein in a cell, wherein the first and the second autofluorescent proteins of the fusion proteins differ from one another in at least one of their detectable photophysical parameters comprising different emission wavelengthes, different fluorescence life times and different polarisations of the emitted light of the autofluorescent proteins,
   b) identifying the second protein that shows a protein-protein-interaction with the first protein by detecting coincidently occurring fluorescence signals of the first and the second autofluorescent proteins in the cells of step a), by measuring the intensities of the individual fluorescence signals of the first and of the second autofluorescent proteins, wherein the fluorescence signals of the first and of the second autofluorescent protein are differentiated from each other either by their different emission wavelengthes, by their different fluorescence life times or by the different polarization of the light emitted by those two autofluorescent proteins.

2. The process of claim 1, wherein the first fusion protein comprises a first protein that is the bait protein and wherein there are more than one second fusion proteins each comprising different second protein which are the prey-proteins.

3. The process of any of claim 1 or 2, wherein the prey-proteins are the individual proteins of a protein library.

4. The process according to claim 1 to 3, wherein the coincidently occurring fluorescence signals of the first and the second autofluorescent proteins in the cells of step a) are determined by comparing the measured fluorescence cross correlation signal with the corresponding cross correlation signal measured for cells, which had been transformed with a negative control vector, indicating 0 % protein-protein-interaction, and by comparing the measured fluorescence cross correlation signal with the corresponding cross correlation signal measured for cells, which had been transformed with a positive control vector, indicating 100 % protein-protein-interaction.

5. The process according to claim 1 to 4, wherein either the first or the second fusion protein has been expressed in the cell by introducing an expression vector comprising the DNA-sequence, coding for said first or said second fusion protein, into the cell by permanent transfection.

6. The process according to claim 1 to 5, wherein either the first or the second fusion protein has been expressed in the cell by introducing an expression vector comprising the DNA-sequence, coding for said first or said second fusion protein, into the cell by transient transfection.

7. The process according to claim 1 to 6, wherein the cell, the first and second fusion protein are expressed in, is selected from HeLa-cells, HEK 293-cells, yeast cells, primary culture cells.

8. The process according to claim 1 to 7, wherein the first and the second fusion protein are expressed under the control of identical promoters.

9. The process according to claim 1 to 7, wherein the first and the second fusion protein are expressed by introducing a single expression vector comprising the DNA-sequences, coding for both, for the first and for the second fusion protein, under the control of the same promoter.

10. The process according to claim 9, wherein the single expression vector comprises an IRES-element or comprises a bidirectional promoter.

11. The process of any of claim 1 to 10, wherein in step a) the first and the second autofluorescent proteins of the fusion proteins differ from each another in their different emission wavelengthes and wherein in step b) the intensities of the individual fluorescence signals of the first and the second autofluorescent proteins in the cells of step a) are detected by measuring the fluorescence signals at these different emission wavelengthes.

12. The process of claim 11, wherein one of the first and the second autofluorescent proteins is a green autofluorescent protein and the other of the first and the second autofluorescent proteins is a red autofluorescent protein.

13. The process of claim 12, wherein the red autofluorescent protein is a dimer or monomer.

14. The process of claim 12 or 13, wherein the red autofluorescent protein is a dimeric or monomeric derivate of DS-RED1 as set force in SEQ ID No. 1.

15. The process of claim 12 to 14, wherein the red autofluorescent protein is a monomer derivate of DS-RED1 as set force in SEQ ID No. 1 comprising non-silent mutations at the positions N42, V44, K163, V175, F177, S197, T217, V71.

16. The process of claim 15, wherein the non-silent mutations are N42Q, V44A, K163M, V175A, F177V, S197I, T217A, V71A.

17. The process of claim 15 or 16, wherein the monomer derivate of DS-RED1 as defined in claim 15 or 16 additionally comprises non-silent mutations at the positions, K83, F124, L150, S179, V195.

18. The process of claim 17, wherein the non-silent mutations are K83L, F124L, L150M, S179T, V195T.

19. The process of claims 15 to 18, wherein the monomer derivate of DS-RED1 additionally comprises non-silent mutations of the positions R2, K5, N6, I125, V127, I180, R153, H162, A164, L174, Y192, Y194, H222, L223, F224, L225.

20. The process of claim 19, wherein the non-silent mutations are R2A, K5E, N6D, I125R, V127T, I180T, R153E, H162K, A164R, L174D, Y192A, Y194K, H222S, L223T, F224G, L225A.

21. The process of any of claims 15 to 20, wherein the red-fluorescent protein is the monomer derivate of DS-RED called mRFP1 as set force in SEQ ID No. 2.

22. The process according to claim 15 to 21, wherein a negative control vector comprising the fusion construct EGFP-IRES-mRFP1 or mRFP-IRES-EGFP, indicating 0 % protein-protein-interaction, is used.

23. The process according to claim 15 to 22, wherein a positive control vector comprising the fusion construct EGFP-mRFP1 or mRFP1-EGFP, indicating 100% protein-protein-interaction is used.

24. The use of two individual autofluorescent proteins, which differ from each another in at least one detectable photophysical parameter, for detecting protein-protein interactions by fluorescence cross correlation spectroscopy (FCCS) in vivo.

25. The use of claim 24, wherein the detectable photophysical parameter which differs between the two individual autofluorescent proteins are either different emission wavelengthes, different fluorescence life times or different polarisations.

26. The use of claim 24 or 25, wherein the two individual autofluorescent proteins differ from each another in their emission wavelengthes and wherein the first autofluorescent protein shows a green fluorescence and the second autofluorescent protein shows a red fluorescence.

27. The use of claim 26, wherein the second autofluorescent protein showing the red fluorescence is a dimer or monomer.

28. The use of claim 26 or 27, wherein the second autofluorescent protein showing the red fluorescence is a dimeric or monomeric derivate of DS-RED1 as set force in SEQ ID No. 1 as defined in any of claims 17 to 23.

Figure 1

Figure 2

Figure 3

Figure 4

Figure 5

Figure 6

Figure 7

Figure 8

Figure 9

Figure 10

**EP 1 645 881 A1**

European Patent Office

**EUROPEAN SEARCH REPORT**

Application Number

EP 04 02 3709

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | BACIA KIRSTEN ET AL: "A dynamic view of cellular processes by in vivo fluorescence auto- and cross-correlation spectroscopy." METHODS (ORLANDO), vol. 29, no. 1, January 2003 (2003-01), pages 74-85, XP008056058 ISSN: 1046-2023 * page 75, column 1, line 8 - line 27 * * page 79, column 2, line 3 - line 32 * * page 84, column 1, paragraph 2 - column 2 * * page 81, column 2, paragraph 4 - page 82, column 1, paragraph 1 * ----- | 1-14, 24-28 | G01N33/58 G01N33/542 |
| X | RUSTOM A ET AL: "ANALYSIS OF FAST DYNAMIC PROCESSES IN LIVING CELLS: HIGH-RESOLUTION AND HIGH-SPEED DUAL-COLOR IMAGING COMBINED WITH AUTOMATED IMAGE ANALYSIS" BIOTECHNIQUES, EATON PUBLISHING, NATICK, US, vol. 28, no. 4, April 2000 (2000-04), pages 722-726,728,73, XP001155803 ISSN: 0736-6205 * page 722, column 3, paragraph 3 - page 723, column 1, paragraph 1 * * figure 2 * * page 730, column 1, paragraph 3 - column 2, paragraph 1 * ----- | 1,5,6,8, 10,11 | |
| X | DITTRICH P ET AL: "ACCESSING MOLECULAR DYNAMICS IN CELLS BY FLUORESCENCE CORRELATION SPECTROSCOPY" BIOLOGICAL CHEMISTRY, vol. 3, no. 382, March 2001 (2001-03), pages 491-494, XP001062907 ISSN: 1431-6730 * page 493, column 1, paragraph 3 - column 2 * ----- -/-- | 1-6,8, 10-14 | TECHNICAL FIELDS SEARCHED (IPC) G01N |

The present search report has been drawn up for all claims

| Place of search | Date of completion of the search | Examiner |
|---|---|---|
| The Hague | 22 November 2005 | Gundlach, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

42

**European Patent Office**

**EUROPEAN SEARCH REPORT**

Application Number

EP 04 02 3709

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | KOHL TOBIAS ET AL: "A protease assay for two-photon crosscorrelation and FRET analysis based solely on fluorescent proteins" PROCEEDINGS OF THE NATIONAL ACADEMY OF SCIENCES OF THE UNITED STATES OF AMERICA, vol. 99, no. 19, 17 September 2002 (2002-09-17), pages 12161-12166, XP008056059 ISSN: 0027-8424 * abstract * | 1,4-14, 24-28 | |
| X | US 2004/014134 A1 (KUHLEMANN RENE ET AL) 22 January 2004 (2004-01-22) * claims 1,4,16,18 * | 1-14, 24-28 | |
| Y | WO 03/086446 A (THE REGENTS OF THE UNIVERSITY OF CALIFORNIA; TSIEN, ROGER, Y; CAMPBELL) 23 October 2003 (2003-10-23) * page 2, line 6 - line 16 * * sequences 4,6,24 * * page 4, line 18 - line 31 * | 15-23 | |
| Y | WO 02/094992 A (RIGEL PHARMACEUTICALS, INCORPORATED; PEELLE, BEAU) 28 November 2002 (2002-11-28) * page 2, line 17 - line 20 * * page 3 * | 15-23 | TECHNICAL FIELDS SEARCHED (IPC) |
| Y | WO 00/34326 A (CLONTECH LABORATORIES, INC; LUKYANOY, SERGEY ANATOLIEVICH; FRADKOV, AR) 15 June 2000 (2000-06-15) N42H * page 33; example 13 * | 15-23 | |

-/--

The present search report has been drawn up for all claims

| Place of search | Date of completion of the search | Examiner |
|---|---|---|
| The Hague | 22 November 2005 | Gundlach, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 04 02 3709

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | CAMPBELL R E ET AL: "A monomeric red fluorescent protein" PROCEEDINGS OF THE NATIONAL ACADEMY OF SCIENCES OF USA, NATIONAL ACADEMY OF SCIENCE, WASHINGTON, DC, US, vol. 99, no. 12, 11 June 2002 (2002-06-11), pages 7877-7882, XP002967837 ISSN: 0027-8424 * figure 1 * | 15-23 | |
| X | WO 01/62919 A (AURORA BIOSCIENCES CORPORATION; NELSON, DAVID; ZAMAIRA, ELIZE; TSIEN,) 30 August 2001 (2001-08-30) * page 43, line 28 - page 45, line 15 * * sequences 7,9 * * claims 1-76 * | 15-23 | |
| A | MEDINA MIGUEL ANGEL ET AL: "Fluorescence correlation spectroscopy for the detection and study of single molecules in biology" BIOESSAYS, vol. 24, no. 8, August 2002 (2002-08), pages 758-764, XP008056112 ISSN: 0265-9247 * the whole document * | 1-28 | TECHNICAL FIELDS SEARCHED (IPC) |

The present search report has been drawn up for all claims

| Place of search | Date of completion of the search | Examiner |
|---|---|---|
| The Hague | 22 November 2005 | Gundlach, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 04 02 3709

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2005

| Patent document cited in search report | | Publication date | Patent family member(s) | | | Publication date |
|---|---|---|---|---|---|---|
| US 2004014134 | A1 | 22-01-2004 | AU | 8398801 | A | 18-02-2002 |
| | | | CA | 2415458 | A1 | 08-01-2003 |
| | | | DE | 10038382 | A1 | 21-02-2002 |
| | | | WO | 0212543 | A2 | 14-02-2002 |
| | | | EP | 1307482 | A2 | 07-05-2003 |
| | | | JP | 2004505636 | T | 26-02-2004 |
| WO 03086446 | A | 23-10-2003 | AU | 2003234702 | A1 | 27-10-2003 |
| | | | CA | 2481328 | A1 | 23-10-2003 |
| | | | EP | 1494697 | A1 | 12-01-2005 |
| | | | JP | 2005522211 | T | 28-07-2005 |
| WO 02094992 | A | 28-11-2002 | NONE | | | |
| WO 0034326 | A | 15-06-2000 | NONE | | | |
| WO 0162919 | A | 30-08-2001 | AU | 3821201 | A | 03-09-2001 |
| | | | CA | 2400642 | A1 | 30-08-2001 |
| | | | EP | 1259608 | A1 | 27-11-2002 |
| | | | JP | 2003523769 | T | 12-08-2003 |

EPO FORM P0459

For more details about this annex : see Official Journal of the European Patent Office, No. 12/82